(19) Europäisches Patentamt
European Patent Office
Office européen des brevets

(11) Veröffentlichungsnummer: **0 669 332 A2**

(12) **EUROPÄISCHE PATENTANMELDUNG**

(21) Anmeldenummer: **95101966.0**

(51) Int. Cl.6: **C07D 471/04**, A01N 43/34

(22) Anmeldetag: **14.02.95**

(30) Priorität: **23.02.94 DE 4405712**

(43) Veröffentlichungstag der Anmeldung: **30.08.95 Patentblatt 95/35**

(84) Benannte Vertragsstaaten: **CH DE FR GB IT LI NL**

(71) Anmelder: **BASF Aktiengesellschaft**
**Carl-Bosch-Strasse 38**
**D-67063 Ludwigshafen (DE)**

(72) Erfinder: **Bratz, Matthias, Dr.**
**Schwabsgasse 2**
**D-67346 Speyer (DE)**
Erfinder: **Meyer, Norbert, Dr.**
**Dossenheimer Weg 22**
**D-68526 Ladenburg (DE)**
Erfinder: **König, Hartmann, Dr.**
**Albert-Einstein-Allee 16**
**D-67117 Limburgerhof (DE)**
Erfinder: **Walter, Helmut, Dr.**
**Grünstadter Strasse 82**
**D-67283 Obrigheim (DE)**
Erfinder: **Gerber, Matthias, Dr.**
**Brandenburger Strasse 24**
**D-67117 Limburgerhof (DE)**
Erfinder: **Westphalen, Karl-Otto, Dr.**
**Mausbergweg 58**
**D-67346 Speyer (DE)**

(54) **Substituierte Naphthyridine und deren herbizide Verwendung.**

(57) Naphthyridine der Formel I,

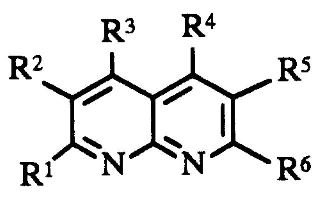

I

in der $R^1$ einen ggf. substituierten Phenylring oder heteroaromatischen Rest darstellt und $R^2$-$R^6$ Wasserstoff, Alkyl, Alkenyl, Cycloalkyl, Halogenalkyl, Alkoxyalkyl, Aminoalkyl, ggf. substituiertes Phenyl, Benzyl, ggf. substituiertes über einen Kohlenstoff verknüpftes Heteroaryl, Cyano, Nitro, Carboxyl, Sulfonylmethyl, Alkoxycarbonyl, Carbamoyl, Alkylcarbamoyl, Dialkylcarbamoyl, Alkanoyl, Benzoyl bedeuten, wobei $R^4$ und $R^5$ oder $R^5$ und $R^6$ durch eine Methylenkette oder durch eine Gruppierung CH = CH-CH = CH verbunden sein können.

EP 0 669 332 A2

Gegenstand der vorliegenden Erfindung sind substituierte Naphthyridine der allgemeinen Formel I

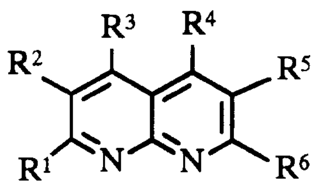

I

in der die Substituenten folgende Bedeutung haben:

$R^1$

(a) ein ggf. substituierter fünfgliedriger Heteroaromat mit ein bis zwei Heteroatomen, ausgewählt aus Stickstoff, Sauerstoff oder Schwefel, wobei der Heteroaromat auch benzokondensiert sein kann;

(b) ein ggf. substituierter sechsgliedriger Heteroaromat enthaltend ein bis zwei Stickstoffatome;

(c) Phenyl, substituiert mit ein bis vier Substituenten ausgewählt aus der Gruppe $C_1$-$C_8$-Alkyl, $C_2$-$C_8$-Alkenyl, $C_2$-$C_8$-Alkinyl, $C_3$-$C_8$-Cycloalkyl, Phenyl, $C_1$-$C_5$-Alkoxy, $C_1$-$C_5$-Thioalkyl, ein fünf -oder sechsgliedriger gesättigter Heterocyclus, ein fünf -oder sechsgliedriger aromatischer Heterocyclus, wobei alle voranstehend genannten Substituenten jeweils noch selber ein- bis dreimal substituiert sein können, Hydroxy, eine Gruppe $OC(O)R^{14}$, Amino, eine Gruppe $NR^{15}R^{16}$, Halogen, Cyano, Nitro, Carboxyl, eine Gruppe $R^7SO_2$-, $-C(O)R^8$, $-C(Y)R^9$, wobei Y für Sauerstoff oder eine Gruppe $NR^{13}$ steht, $-C(VR^{10}WR^{11})-R^{12}$, wobei V und W unabhängig voneinander für O oder S stehen;

- $R^7$ $C_1$-$C_8$-Alkyl, $C_3$-$C_8$-Cycloalkyl, $C_1$-$C_8$-Halogenalkyl, $C_1$-$C_5$-Alkoxy, Phenyl, Naphthyl, Aryl-($C_1$-$C_6$-)alkyl, Heteroaryl, Hydroxy, Amino, $C_1$-$C_8$-Monoalkyl- oder $C_1$-$C_8$-Dialkylamino, $C_5$-$C_8$-Cycloalkylamino;
- $R^8$ Hydroxy, Amino, $C_1$-$C_8$-Monoalkyl- oder $C_1$-$C_8$-Dialkylamino, $C_5$-$C_8$-Cycloalkylamino, $C_1$-$C_6$-Alkylthio, $C_1$-$C_8$-Alkoxy;
- $R^9$ Wasserstoff, $C_1$-$C_8$-Alkyl, $C_3$-$C_8$-Cycloalkyl, $C_1$-$C_8$-Halogenalkyl, $C_1$-$C_4$-Alkoxy($C_1$-$C_6$-)alkyl, $C_1$-$C_8$-Aininoalkyl, Phenyl, Aryl($C_1$-$C_6$-)alkyl, Heteroaryl;
- $R^{10}$ $R^{11}$ $C_1$-$C_8$-Alkyl, $C_1$-$C_6$-Halogenalkyl, $C_1$-$C_4$-Alkoxy($C_1$-$C_6$-)alkyl oder $R^{10}$ und $R^{11}$ bilden zusammen eine Di-, Tri-, oder Tetramethylenkette die ggf. durch ein bis zwei $C_1$-$C_8$-Alkyl-, $C_1$-$C_6$-Halogenalkyl-, $C_1$-$C_4$-Alkoxy($C_1$-$C_6$-)alkylgruppen oder durch eine Oxogruppe substituiert ist;
- $R^{12}$ Wasserstoff, $C_1$-$C_8$-Alkyl, $C_3$-$C_8$-Cycloalkyl, $C_1$-$C_8$-Halogenalkyl, $C_1$-$C_4$-Alkoxy($C_1$-$C_6$-)alkyl, $C_1$-$C_8$-Aminoalkyl, Phenyl, Aryl($C_1$-$C_6$-)alkyl, Heteroaryl;
- $R^{13}$ $C_1$-$C_8$-Alkyl, $C_2$-$C_8$-Alkenyl, $C_3$-$C_8$-Cycloalkyl, $C_1$-$C_8$-Halogenalkyl, $C_1$-$C_4$-Alkoxy($C_1$-$C_6$-)alkyl, $C_1$-$C_8$-Aminoalkyl, Phenyl, Aryl($C_1$-$C_6$-)alkyl, Heteroaryl, Hydroxy, ggf. substituiertes $C_6$-$C_{10}$-Aryloxy, ggf. substituiertes $C_1$-$C_8$-Alkoxy, Amino, $C_1$-$C_8$-Monoalkyl- oder $C_1$-$C_8$-Dialkylamino, $C_5$-$C_8$-Cycloalkylamino, ggf substituiertes $C_6$-$C_{10}$-Arylamino;
- $R^{14}$ $C_1$-$C_5$-Alkyl, ggf. substituiertes Phenyl, $C_1$-$C_5$-Alkoxy, $C_1$-$C_5$-Halogenalkoxy, Benzyloxy, Amino, Alkylamino, Dialkylamino, ein gesättigter über N gebundener fünf- oder sechsgliedriger Heterocyclus, der noch ein weiteres Heteroatom N oder O enthalten kann;
- $R^{15}$ Wasserstoff, $C_1$-$C_5$-Alkyl, ggf. substituiertes Phenyl;
- $R^{16}$ Wasserstoff, $C_1$-$C_5$-Alkyl, ggf. substituiertes Phenyl, eine Gruppe $-C(O)R^{14}$ oder $R^{16}$ bildet gemeinsam mit $R^{15}$ eine vier- oder fünfgliedrige Methylenkette, die ihrerseits mit ein oder zwei Methylgruppen substituiert sein kann und in der eine Methylengruppe durch Sauerstoff ersetzt sein kann;

(d) Phenyl, in dem zwei direkt benachbarte Positionen über eine Tri- oder Tetramethylengruppe, welche ihrerseits ein bis drei $C_1$-$C_3$-Alkyl-, $C_1$-$C_2$-Alkoxy- und/oder Halogensubstituenten tragen kann, miteinander verbunden sind und die übrigen Positionen unsubstituiert oder durch einen der unter (c) aufgeführten Phenylsubstituenten substituiert sind;

(e) Phenyl, das über die ortho-Position mit $R^2$ verbunden ist zu einer Kette aus ein bis vier Methylengruppen oder ein bis drei Methylengruppen und einem Sauerstoffatom, wobei jede Methylengruppen ein oder zwei $C_1$-$C_3$-Alkylreste tragen kann;

$R^2$-$R^6$

Wasserstoff, $C_1$-$C_8$-Alkyl, $C_2$-$C_8$-Alkenyl, $C_3$-$C_8$-Cycloalkyl, $C_1$-$C_8$-Halogenalkyl, $C_1$-$C_4$-Alkoxy($C_1$-$C_6$)-alkyl, $C_1$-$C_8$-Aminoalkyl, ggf. substituiertes Phenyl, Benzyl, ggf substituiertes über einen Kohlenstoff verknüpftes fünf- oder sechsgliedriges Heteroaryl, Cyano, Nitro, Carboxyl, Sulfonylmethyl, $C_1$-$C_4$-Alkoxycarbonyl, Carba-

moyl, $C_1$-$C_8$-Alkylcarbamoyl, Dialkylcarbamoyl, $C_1$-$C_4$-Alkanoyl, Benzoyl oder $R^4$ und $R^5$ oder $R^5$ und $R^6$ bilden gemeinsam eine Kette $CH_2$-$(CH_2)_n$-$CH_2$ mit n = oder einen unkondensierten aromatischen Ring; mit der Maßgabe, daß Verbindungen mit folgenden Substituentenkombinationen ausgenommen sind:

i) wenn $R^1$ = Phenyl und $R^3$-$R^6$ = Wasserstoff, steht $R^2$ nicht für Wasserstoff, Methyl, Ethyl, n-Propyl, Phenyl oder Chlor;

ii) wenn $R^1$ = Phenyl und $R^2$-$R^5$ = Wasserstoff; steht $R^6$ nicht für Phenyl oder Methyl;

iii) wenn $R^1$ = Phenyl und $R^2$-$R^4$ sowie $R^6$ = Wasserstoff, steht $R^5$ nicht für Phenyl oder Benzoyl;

iv) wenn $R^1$ = Phenyl, $R^2$-$R^4$ = Wasserstoff und $R^6$ = Methyl, steht $R^5$ nicht für Acetyl oder Ethoxycarbonyl;

v) wenn $R^1$ = Phenyl, $R^3$ = Chlor, $R^2$; $R^4$ und $R^5$ = Wasserstoff, steht $R^6$ nicht für Methyl;

vi) wenn $R^1$ = Phenyl, $R^2$ = Cyano und $R^3 + R^5$ = Wasserstoff, stehen $R^4$ und $R^6$ nicht gleichzeitig für Methyl;

vii) wenn $R^2$-$R^6$ Wasserstoff,

steht $R^1$ nicht für 2-Pyridyl, 3-Pyridyl, 4-Pyridyl, 2-Thienyl, 2-Furanyl, 1-Naphthyl, 2-Naphthyl, Phenyl, 4-Chlorphenyl, 4-Bromphenyl, 4-Nitrophenyl, 4-Hydroxyphenyl, 4-Methoxyphenyl, 4-Methylphenyl, p-Biphenyl, 2-Hydroxy-4-chlorphenyl, 3-Nitrophenyl, 3-Trifluormethylphenyl, 2-Hydroxyphenyl, 2-Methoxyphenyl, 2-Hydroxy-5-nitrophenyl, 2-Hydroxy-5-methylphenyl;

viii) 6-H-Indeno-[1,2-b][1.8]-naphthyridin, 5,6-Dihydronaphto[1.2-b][1.8]naphthyridin, 6H-[1]benzopyrano-[4.3-b][1.8]naphthyridin oder 6,7-Dihydro-5H-benzo[6.7]cyclohepta[1,2-b][1.8]naphthyridin.

Gegenstand der Erfindung sind ferner herbizide Mittel sowie Mittel zur Bekämpfung von Schadpilzen, enthaltend Naphthyridine der Formel I sowie Verfahren zur Bekämpfung unerwünschten Pflanzenwuchses oder zur Bekämpfung von Schadpilzen mit den Naphthyridinen der Formel I, einschließlich der über Disclaimer vom Stoff ausgenommenen Einzelverbindungen.

Naphthyridine mit unterschiedlichsten Substitutionsmustern sind seit langem in der Chemie bekannt und haben die verschiedensten Anwendungen gefunden. Ein Großteil der beschriebenen Verbindungen ist dadurch gekennzeichnet, daß in verschiedenen Positionen des Rings eine Amino- oder Hydroxyfunktion bzw. Derivate davon vorhanden sind. Die biologische Wirkung solcher Verbindungen reicht von verschiedenen pharmakologischen (z. B. EP-A 346 208, EP-A 391 185, J. Med. Chem. 1977, 20 838 über fungizide (EP-A 410 762) bis hin zu herbiziden Anwendungen (z. B. EP 329 012, WO 92/07468). Integraler Bestandteil all dieser Verbindungen sind über Heteroatome (Stickstoff, Sauerstoff oder Schwefel) an das Naphthyridin gebundene Gruppen.

Seltener bekannt geworden sind Daten über die biologische Wirksamkeit von Verbindungen der Formel I, in der $R^1$ beispielsweise ein ggf. substituierter Phenylring ist und die Reste $R^2$ - $R^6$ nicht die oben angeführte Bedeutung (OH, $NH_2$, $NR_2$) haben.

Lediglich Reddy et al. (Ind. J. Chem. 1988, 27B, 200-202; J. Indian Chem. 1986, 443 - 446) berichten über eine schwache antibakterielle Aktivität von Verbindungen des Typs I mit $R^1$ = substituiertes Phenyl. Einzelne Naphthyridine vom Typ I sind ferner bekannt aus:

E.M. Hawes, D.G. Wibberley, J. Chem. Soc.(C) 1967, 1564 und loc. cit. 1966, S. 315-320; T. Higashino et al., Chem. Pharm. Bull 1978, 26, 3242; Yakugaku Zasshi 1979, 99, 451-7 (CA 92(19): 163866a); D.K. Garecki, E.M. Hawes, J. Med. Chem. 1977, 20(1), 124 und loc. cit. 20(6), Seiten 838-840; J.P. Sauvage et al., Tetrahedron Lett. 1982, 23, 5291; R.P. Thummel et al., J. Heterocycl. Chem. 1986, 23, 689; E.J.S. Reddy et al., Natl. Acad. Sci. Lett. (India) 1985, 8, 19 (CA 103: 51079k);

Der Erfindung lag die Aufgabe zugrunde, Naphthyridine zu finden, die eine gute herbizide Wirkung aufweisen. Demgemäß wurde gefunden, daß Verbindungen des Typs I starke herbizide Wirkung gegen Unkräuter und Gräser im Vorauflauf- und Nachauflaufverfahren aufweisen. Ferner wurde gefunden, daß Verbindungen **I** wichtige Kulturpflanzen nicht, oder nur unwesentlich schädigen und so zu einer selektiven Bekämpfung von Schadpflanzen in diesen Kulturen geeignet sind. Zu diesen Kulturen gehören beispielsweise Soja, Baumwolle, Weizen, Reis und Mais.

Die erfindungsgemäßen Verbindungen I lassen sich nach einer Vielzahl von Methoden herstellen (siehe z. B. Katrizky, Comprehensive Heterocyl. Chem. Vol 2, p. 607 ff).

Als besonders geeignet zur Herstellung von I erwies sich die Herstellung der Verbindungen nach den folgenden Verfahren:

a) Kondensation aromatischer und heteroaromatischer Ketone mit 2-Amino-nikotinaldehyden (Friedländer Synthese, z. B. Hawes und Wibberley, J. Chem. Soc. 1966, *315;* Caluwe, Tetrahedron 1980, *36, 2359–2407).*

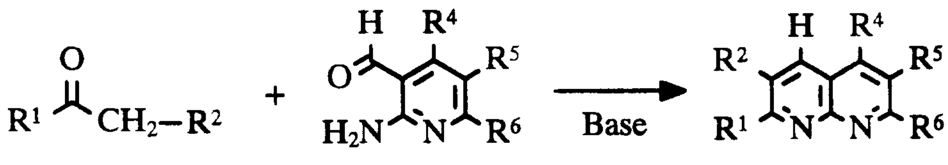


II III I

b) Analog hierzu lassen sich auch entsprechende Aminoketone einsetzen.

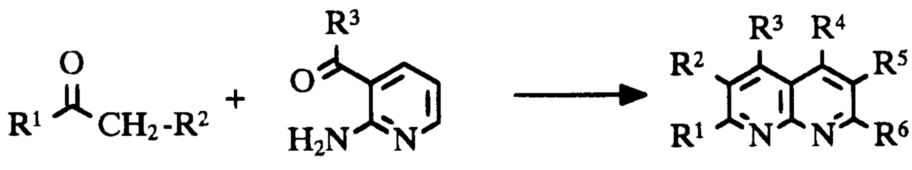

II IIIa I

Die Umsetzung methylenaktiver Ketone mit 2-Amino-nikotinaldehyden oder -ketonen erfolgt vorzugsweise in einem Lösungs- oder Verdünnungsmittel, beispielsweise in Wasser, in einem Alkohol wie Methanol, Ethanol, Propanol, Isopropanol oder Ethoxyethanol, in flüssigem Ammoniak, in einem Ether wie Tetrahydrofuran oder Dioxan, in einem aromatischen Kohlenwasserstoff wie Benzol, Toluol, Chlorbenzol und Nitrobenzol, in einem polaren aprotischen Lösungsmittel wie Acetonitril, Dimethylformamid, Dimethylsulfoxid und N-Methylpyrrolidon oder in Gemischen der genannten Lösungsmittel.

Vorteilhaft führt man die Umsetzungen in Gegenwart einer organischen oder anorganischen Base durch, wobei z. B. die Hydroxide, Hydride, Alkoxide, Amide, Carbonate und Hydrogencarbonate der Alkali- und Erdalkalimetalle in Betracht kommen. Besonders eignen sich Alkalimetallhydroxide wie Lithiumhydroxid, Natriumhydroxid und Kaliumhydroxid, Erdalkalimetallhydroxide wie Bariumhydroxid und Calciumhydroxid, Alkalimetallhydride wie Natriumhydrid und Kaliumhydrid, Erdalkalimetallhydride wie Calciumhydrid, Alkalimetallalkoholate wie Natriummethanolat, Natriumethanolat und Kalium-tert.-butylat, Alkalimetallamide wie Natriumamid und Lithium-diisopropylamid, Alkalimetallcarbonate und -hydrogencarbonate wie Natriumbicarbonat, Natriumhydrogencarbonat, Kaliumbicarbonat und Kaliumhydrogenoarbonat. Unter den organischen Basen sind aliphatische Amine wie Triethylamin, Dimethylamin, Diethylamin und Diisopropylamin, cycloaliphatische Amine wie Piperidin, Morpholin, Pyrrolidin, DBU und DABCO sowie aromatische Amine wie Pyridin, N,N-Dimethylaminopyridin und Chinolin besonders bevorzugt.

Verwendet man ein Amin als Base, so kann auch lösungsmittelfrei in einem Überschuß der Base gearbeitet werden.

Zweckmäßigerweise setzt man die Edukte II und III in etwa stöchiometrischem Verhältnis ein oder man arbeitet mit einem Überschuß an Methylenverbindung II bis ca. 100 mol-%, bezogen auf III.

Die Menge an Base ist nicht kritisch. Sie beträgt in der Regel 10-50 mol-%, kann jedoch auch im Überschuß eingesetzt werden.

Bei Verwendung einer organischen Base kann ohne Lösungsmittel in einem Überschuß an Base, bis zur etwa 10fachen molaren Menge, bezogen auf das Aminopyridin III, gearbeitet werden.

Im allgemeinen liegt die Reaktionstemperatur zwischen 0 und 200 °C, bevorzugt zwischen 20 und 150 °C, insbesondere bei etwa 20-30 °C (Raumtemperatur) oder bei der Siedetemperatur des jeweiligen Lösungsmittels.

Weiterhin ist es möglich, die Reaktion in Gegenwart einer organischen oder anorganischen Säure durchzuführen. Hierfür kommen in Frage Ameisensäure, Essigsäure, Propionsäure, Pivalinsäure, Salzsäure, Bromwasserstoffsäure, Schwefelsäure, Phosphorsäure, Salpetersäure oder Gemische dieser Säuren beispielsweise Essigsäure und Schwefelsäure. Die Säuren können ggf. mit Wasser verdünnt werden. Weiterhin kann es vorteilhaft sein, die Säure als Lösemittel einzusetzen.

c) Als Substitut der empfindlichen Aminoaldehyde lassen sich beispielsweise Pyrido[2,3d]pyrimidine einsetzen (Higashino et al., Chem. Pharm. Bull 1978, *28*, 3242-3244).

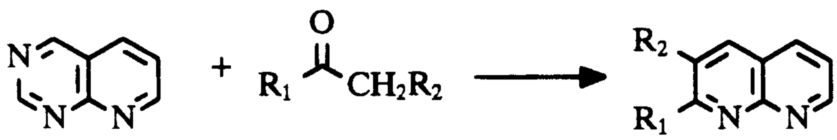

d) Umgekehrt lassen sich die Reste $R^1$, $R^2$ und $R^3$ auch aus dem Nikotinaldehyd heraus einführen:

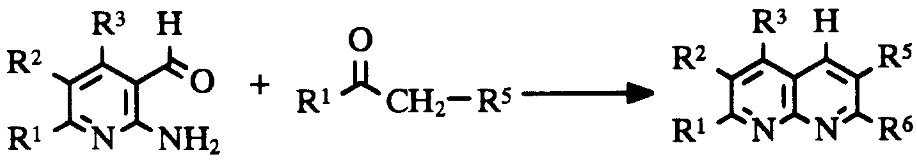

e) In einigen Fällen führt die basenkatalysierte Kondensation zu einem intermediären Aminoketon:

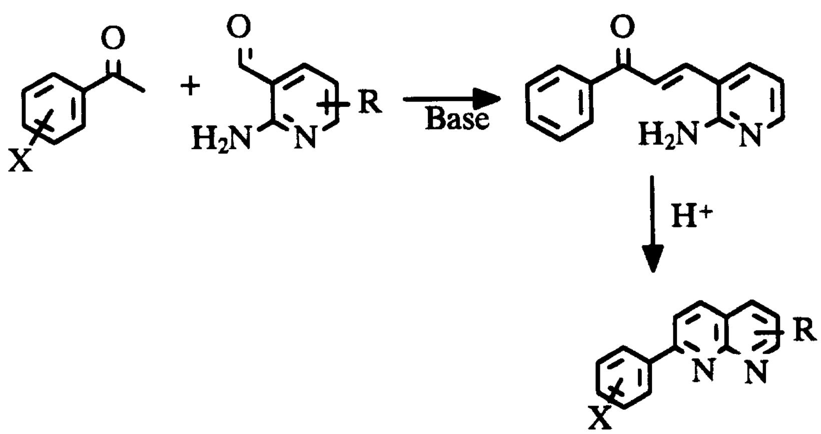


welches nicht weiterreagiert und isoliert werden kann. Die Verbindungen können dann in Gegenwart von Säure (z. B. $CH_3COOH/H_2SO_4$) zum gewünschten Naphthyridin geschlossen werden.

f) Ausgehend von substituierten Aminopyridinen lassen sich die Naphthyridine nach Art der Skraupschen-Synthese durch Umsetzung von $\alpha,\beta$-ungesättigten Aldehyden und Ketonen erhalten:

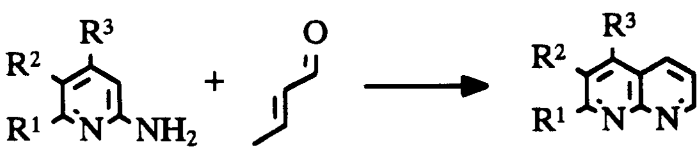

Die Synthese der für die Friedländer Synthese benötigten Aminonikotinaldehyde erfolgt nach allgemein bekannten Verfahren:

a) Aus 2-Halogen-3-cyanopyridinen

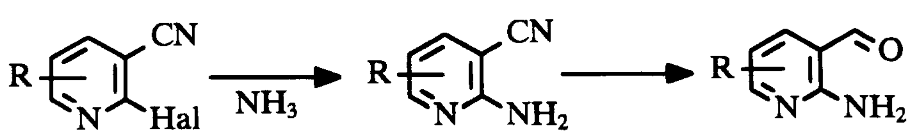


b) Aus Pyrido[2,3d]pyrimidinen (Evans und Caluwe J. Org. Chem. 1975, 40, 1438-1439; Caluwe, J. Org. Chem. 1974, 39, 720-21)

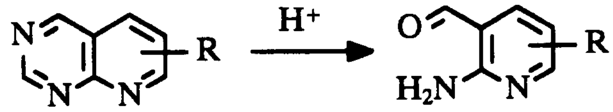


c) Aus 2-Amino-3-methylpyridinen (Hagen et al. DE-A 36 14 698)

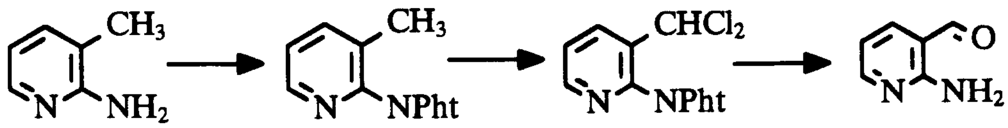

Pht = Phthaloyl

d) Umsetzung von 1,3-Butadien-1,1-dicarbonitrilen mit $NH_3$ und anschließender Reduktion:

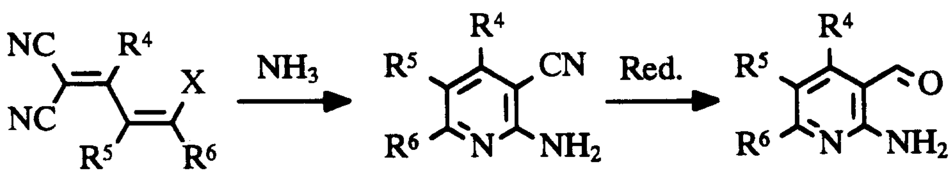


$X = OR, NR^2$

(z. B. DE-A 41 17 802)

Die eingesetzten Ketone sind meist kommerziell erhältlich, literaturbekannt oder lassen sich in allgemein bekannter Weise darstellen.

In Formel I können die genannten Reste zum Teil substituiert sein. Sofern nachstehend nicht im einzelnen konkretisiert, bedeutet der Ausdruck "ggf. substituiert" eine $C_1$-$C_4$-Alkylgruppe, $C_1$-$C_4$-Alkoxy, $C_1$-$C_4$-Halogenalkyl, $C_1$-$C_4$-Halogenalkoxy oder Halogen wie insbesondere Fluor, Chlor oder Brom. Der Ausdruck "Halogen" steht für Fluor, Chlor, Brom oder Iod, insbesondere Fluor und Chlor. Fünf- oder sechsgliedrige gesättigte Heterocyclen sind z.B. Piperidyl, Pyrolidinyl, Tetrahydrofuranyl, Tetrahydropyranyl, Piperazinyl oder Morpholino. "Aryl" allein oder in Kombination bedeutet Phenyl oder Naphthyl, Heteroaryl, Thienyl, Furanyl, Pyrrolyl, Thiazolyl, Oxazolyl, Pyridyl, Imidazolyl, 1,24-Triazolyl oder Osoxazolyl.

Im Hinblick auf die biologische Wirkung sind Naphthyridine der Formel I besonders bevorzugt, wenn $R^1$ die folgende Bedeutung hat:

Phenyl unsubstituiert oder substituiert mit ein bis vier, insbesondere ein oder zwei Substituenten, ausgewählt aus der Gruppe

$C_1$-$C_8$-Alkyl, insbesondere $C_1$-$C_4$-Alkyl wie Methyl oder Ethyl, ggf. substituiert mit Halogen, $C_1$-$C_4$-Alkoxy, Amino, $C_1$-$C_4$-Alkylamino, $C_1$-$C_4$-Dialkylamino, $C_1$-$C_4$-Thioalkyl, Phenyl, Phenoxy, Thiophenyl, Aminophenyl, $C_1$-$C_4$-Alkylaminophenyl, wobei die fünf letztgenannten Substituenten im Phenylring ein bis drei Substituenten, ausgewählt aus $C_1$-$C_4$-Alkoxy, Halogen, $C_1$-$C_4$-Alkyl oder Nitro tragen können;

$C_2$-$C_8$-Alkenyl oder -Alkinyl, jeweils bevorzugt mit bis zu vier C-Atomen und ggf. substituiert mit Halogen, $C_1$-$C_4$-Alkoxy, $C_1$-$C_4$-Thioalkyl, Phenyl, Phenoxy, Thiophenyl, wobei die drei letztgenannten Substituenten im Phenylring ein bis drei Substituenten, ausgewählt aus $C_1$-$C_4$-Alkoxy, Halogen, $C_1$-$C_4$-Alkyl oder Nitro tragen können;

$C_3$-$C_8$-Cycloalkyl, ggf. substituiert mit Halogen, $C_1$-$C_4$-Alkoxy, Amino, $C_1$-$C_4$-Alkylamino, $C_1$-$C_4$-Dialkylamino, $C_1$-$C_4$-Thioalkyl, Phenyl, Phenoxy, Thiophenyl, Aminophenyl, $C_1$-$C_4$-Alkylaminophenyl, wobei die fünf letztgenannten Substituenten im Phenylring ein bis drei Substituenten, ausgewählt aus $C_1$-$C_4$-Alkoxy, Halogen, $C_1$-$C_4$-Alkyl oder Nitro tragen können;

Phenyl ggf. substituiert mit bis zu drei Substituenten, ausgewählt aus $C_1$-$C_4$-Alkoxy, Halogen, $C_1$-$C_4$-Alkyl oder Nitro;

$C_1$-$C_5$-Alkoxy, ggf. substituiert mit Halogen, $C_1$-$C_4$-Alkoxy, Cyano, $C_1$-$C_4$-Dialkylamino, $C_1$-$C_4$-Thioalkyl, Phenyl, Phenoxy, Thiophenyl, Aminophenyl, $C_1$-$C_4$-Alkylaminophenyl, wobei die fünf letztgenannten Substituenten im Phenylring ein bis drei Substituenten, ausgewählt aus $C_1$-$C_4$-Alkoxy, Halogen, $C_1$-$C_4$-Alkyl oder Nitro tragen können;

$C_1$-$C_5$-Thioalkyl, ggf. halogensubstituiert, z.B. $C_1$-$C_4$-Halogenthioalkyl wie Chlorthiomethyl oder Dichlorthiomethyl;

ein fünf- oder sechsgliedriger gesättigter Heterocyclus mit ein bis zwei Heteroatomen N, O oder S wie z.B. Thienyl, Furanyl, Pyrrolyl, Thiazolyl, Oxazolyl, Isooxazolyl, Imidazolyl, 1,2,4-Triazolyl, Isothiazolyl, 1,2,4-Oxadiazolyl oder 1,3,4-Thiadiazolyl;

oder ein fünf- oder sechsgliedriger Heteroaromat mit ein bis drei Stickstoffatomen oder einem Stickstoffatom und einem weiteren O- oder S-Atom oder mit nur einem Heteroatom Sauerstoff oder Schwefel wie beispielsweise Pyridyl, Pyrimidyl, Pyrazinyl, Triazinyl;

wobei die genannten heterocyclischen oder heteroaromatischen Reste, die über ein C-Atom oder ein N-Atom gebunden sind, jeweils ggf. 1- bis 3-fach, insbesondere 1-fach substituiert sind, durch $C_1$-$C_4$-Alkoxy, Halogen, $C_1$-$C_4$-Alkyl, Cyano, Hydroxy, Amino, $C_1$-$C_4$-Alkylamino, $C_1$-$C_4$-Dialkylamino, $C_1$-$C_4$-Alkoxycarbonyl oder Nitro;

ferner der Phenylrest $R^1$ substituiert sein kann durch: Hydroxy, eine Gruppe $OC(O)R^{14}$, Amino, eine Gruppe $NR^{15}R^{16}$, Halogen wie Fluor, Chlor oder Brom, Cyano, Nitro, Carboxyl, eine Gruppe $R^7SO_2$,$C(O)R^8$, $C(Y)R^9$ mit $Y = O$ oder $NR^{13}$, eine Gruppe $C(VR^{10}WR^{11})R^{12}$ mit V und W unabhängig voneinander Sauerstoff oder Schwefel,

und $R^7$-$R^{16}$ bedeuten:

| | |
|---|---|
| $R^7$ | $C_1$-$C_8$-Alkyl, $C_1$-$C_8$-Halogenalkyl, Phenyl, $C_1$-$C_8$-Monoalkyl- oder $C_1$-$C_8$-Dialkylamino, $C_5$-$C_8$-Cycloalkylamino; |
| $R^8$ | Hydroxy, Amino, $C_1$-$C_8$-Monoalkyl- oder $C_1$-$C_8$-Dialkylamino, $C_5$-$C_8$-Cycloalkylamino, $C_1$-$C_8$-Alkoxy; |
| $R^9$ | Wasserstoff, $C_1$-$C_8$-Alkyl, $C_3$-$C_8$-Cycloalkyl, $C_1$-$C_8$-Halogenalkyl, Phenyl, Heteroaryl; |
| $R^{10}$, $R^{11}$ | $C_1$-$C_8$-Alkyl, $C_1$-$C_6$-Halogenalkyl, $C_1$-$C_4$-Alkoxy($C_1$-$C_6$-)alkyl oder $R^{10}$ und $R^{11}$ bilden zusammen eine Di-, Tri-, oder Tetramethylenkette, die ggf. durch ein bis zwei $C_1$-$C_8$-Alkyl-, $C_1$-$C_6$-Halogenalkyl-, $C_1$-$C_4$-Alkoxy ($C_1$-$C_6$-)alkylgruppen oder eine Oxogruppe substituiert ist; |
| $R^{12}$ | Wasserstoff, $C_1$-$C_8$-Alkyl, $C_3$-$C_8$-Cycloalkyl, $C_1$-$C_8$-Halogenalkyl, Phenyl, Heteroaryl; |
| $R^{13}$ | $C_1$-$C_8$-Alkyl, $C_3$-$C_8$-Cycloalkyl, $C_1$-$C_4$-Alkoxy($C_1$-$C_6$-)alkyl, $C_1$-$C_8$-Aminoalkyl, Phenyl, Aryl-($C_1$-$C_6$-)alkyl, Heteroaryl, Hydroxy, ggf. substituiertes $C_6$-$C_{10}$-Aryloxy, ggf. substituiertes $C_1$-$C_8$-Alkoxy, Amino, $C_1$-$C_8$-Monoalkyl- oder $C_1$-$C_8$-Dialkylamino, $C_5$-$C_8$-Cycloalkylamino, ggf substituiertes $C_6$-$C_{10}$-Arylamino; |
| $R^{14}$ | $C_1$-$C_5$-Alkyl, ggf. subst. Phenyl, $C_1$-$C_5$-Alkoxy, $C_1$-$C_5$-Halogenalkoxy, Benzyloxy, Amino, Alkylamino, Dialkylamino, ein gesättigter über N gebundener fünf- oder sechsgliedriger Heterocyclus; |
| $R^{15}$ | Wasserstoff, $C_1$-$C_5$-Alkyl, ggf. substituiertes Phenyl; |
| $R^{16}$ | Wasserstoff, $C_1$-$C_5$-Alkyl, ggf. sübstituiertes Phenyl, eine Gruppe $-C(O)R^{14}$ oder $R^{16}$ bildet gemeinsam mit $R^{15}$ eine vier- oder fünfgliedrige Methylenkette, die ihrerseits mit ein bis zwei Methylgruppen substituiert sein kann und in der eine Methylengruppe durch Sauerstoff ersetzt sein kann; |

$R^1$ ferner bevorzugt ein Phenylrest, in dem zwei direkt benachbarte Positionen über eine Tri- oder Tetramethylengruppe, welche ihrerseits ein bis drei $C_1$-$C_3$-Alkyl-, $C_1$-$C_2$-Alkoxy- und/oder Halogensubstituenten tragen kann, miteinander verbunden sind und die übrigen Positionen unsubstituiert oder durch einen der voranstehend aufgeführten Substituenten an Phenylring, insbesondere durch $C_1$-$C_4$-Alkyl, z.B. Methyl, Ethyl, i-Propyl, n-Propyl, n-Butyl, i-Butyl substituiert;

$R^1$ ferner Phenyl, das über die ortho-Position mit $R^2$ verbunden ist zu einer Kette aus ein bis vier Methylengruppen oder ein bis drei Methylengruppen und einem Sauerstoffatom, wobei jede Methylengruppen ein oder zwei $C_1$-$C_3$-Alkylreste tragen kann, z.B. zu einer Kette $-CH_2-CH_2-CH_2-$, $-CH_2-CH_2-CH_2-CH_2-$, $-CH_2-O-CH_2-CH_2-$, $-CH_2(CH_3)-CH_2-CH_2-CH_2-$, $-CH_2-O-CH_2-$, $-O-CH_2-CH_2-$, $-C(CH_3)_2-CH_2-CH_2-$,$-CH(CH_3)-CH_2-CH_2-$.

$R^1$ ferner ein Heteroaromat, ausgewählt aus der Gruppe aus 2-Thienyl, 2-Benzothienyl, 3-Thienyl, 2-Furanyl, 3-Furanyl, 2-Benzofuranyl, 2-Pyrrolyl, 3-Pyrrol, 2-Thiazolyl, 4-Thiazolyl, 5-Thiazolyl, 2-Oxazolyl, 4-Oxazolyl, 5-Oxazolyl, 2-Pyridyl, 3-Pyridyl, 4-Pyridyl, 2-Pyrimidinyl, 4-Pyrimidinyl, 5-Byrimidinyl, 2-Benzothiazolyl, 2-Benzooxazolyl, besonders bevorzugt 2- oder 3-Thienyl, wobei die Heteroaromaten ein bis zwei der folgenden Substituenten tragen können:

$C_1$-$C_8$-Alkyl, $C_3$-$C_8$-Cycloalkyl, $C_1$-$C_8$-Halogenalkyl, Phenyl, $C_1$-$C_5$-Alkoxy, $C_1$-$C_3$-Halogenalkoxy, Halogen, Cyano, Nitro, Carboxyl, Sulfonylmethyl, $C_1$-$C_4$-Alkoxycarbonyl, Carbamoyl, $C_1$-$C_4$-Alkylcarbamoyl, Di-($C_1$-$C_4$)-alkylcarbamoyl.

Die Reste $R^2$-$R^6$ in Formel I bedeuten beispielsweise Wasserstoff, $C_1$-$C_8$-Alkyl, insbesondere $C_1$-$C_4$-Alkyl wie Methyl, Ethyl oder Propyl, $C_2$-$C_8$-Alkenyl, insbesondere $C_2$-$C_4$-Alkenyl wie Vinyl oder Allyl, $C_3$-$C_8$-Cycloalkyl, insbesondere Cyclopentyl, Cyclohexyl oder Cyclopropyl, $C_1$-$C_8$-Halogenalkyl, z.B. $C_1$-$C_4$-Halogenalkyl wie Trifluormethyl, Trichlormethyl, Fluorethyl oder Chlorethyl, $C_1$-$C_4$-Alkoxy($C_1$-$C_6$)-alkyl wie Methoxymethyl, Methoxyethyl, Ethoxymethyl oder Ethoxyethyl, $C_1$-$C_8$-, insbesondere $C_1$-$C_4$-Aminoalkyl wie Aminomethyl oder Aminoethyl, Phenyl oder Benzyl, jeweils ggf. substituiert insbesondere durch ein bis drei Reste wie $C_1$-$C_4$-Alkyl, Halogen, $C_1$-$C_4$-Halogenalkyl, $C_1$-$C_4$-Alkoxy oder Nitro; über ein C-Atom verknüpftes Heteroaryl, z.B. Pyridyl, Thienyl oder Furanyl, wobei der Heteroaromat substituiert sein kann, z.B. durch Methyl, Chlor, Nitro oder Cyano;

$R^2$-$R^6$ ferner Cyano, Nitro, Carboxyl, Sulfonylmethyl, $C_1$-$C_4$-Alkoxycarbonyl, Carbamoyl, $C_1$-$C_4$-Alkylcarbamoyl, Di($C_1$-$C_4$-)-alkylcarbamoyl, $C_1$-$C_4$-Alkanoyl wie Acetyl, Benzoyl.

Ferner können zwei benachbarte Reste $R^4$ und $R^5$ oder $R^5$ und $R^6$ gemeinsam eine Kette $-CH_2-(CH_2)_n-CH_2$ mit n = 1 bis 3 bilden oder einen ankondensierten aromatischen Ring, d.h. zusammen eine Brücke -CH = CH-CH = CH-.

Die erfindungsgemäßen Verbindungen zeichnen sich dadurch aus, daß eine besonders starke herbizide Wirkung auftritt, wenn:

- $R^1$ die Bedeutung Phenyl hat und mindestens ein Substituent in der ortho-Position des Phenylringes steht;
- $R^1$ die Bedeutung unsubstituiertes Phenyl hat, der Substituent $R^2$ nicht die Bedeutung Wasserstoff hat.

Wenn der Substituent $R^1$ die Bedeutung substituiertes Phenyl hat, sind folgende Bedeutungen als besonders bevorzugt anzusehen:

Phenyl substituiert mit:

2-Methyl, 3-Methyl, 4-Methyl, 2-Ethyl, 3- Ethyl, 4-Ethyl, 2-Propyl, 3-Propyl, 4-Propyl, 2-i-Propyl, 3-i-Propyl, 4-i-Propyl, 2-n-Butyl, 3-n-Butyl, 4-n-Butyl, 2-s-Butyl, 3-s-Butyl, 4-s-Butyl, 2-t-Butyl, 3-t-Butyl, 4-t-Butyl, 2-Benzyl, 3-Benzyl, 4-Benzyl, 2-cyclo-Propyl, 3-cyclo-Propyl, 4-cyclo-Propyl, 2-Trifluomethyl, 3-Trifluormethyl, 4-Trifluormethyl, 2-Methoxy, 3-Methoxy, 4-Methoxy, 2-Ethoxy, 3-Ethoxy, 4-Ethoxy, 2-Trifluormethoxy, 3-Trifluormethoxy, 4-Trifluormethoxy, 2-Thiomethyl, 3-Thiomethyl, 4-Thiomethyl, 2-Fluor, 3-Fluor, 4-Fluor, 2-Chlor, 3-Chlor, 4-Chlor, 2-Brom, 3-Brom, 4-Brom, 2-Nitro, 3-Nitro, 4-Nitro, 2-Cyano, 3-Cyano, 4-Cyano, 2-Carbomethoxy, 3-Carbomethoxy, 4-Carbomethoxy, 2-Carboethoxy, 3-Carboethoxy, 4-Carboethoxy, 2-Carbamoyl, 3-Carbamoyl, 4-Carbamoyl, 2-N-Methylcarbamoyl, 3-N-Methylcarbamoyl, 4-N-Methylcarbamoyl, 2-N,N-Dimethylcarbamoyl, 3-N,N-Dimethylcarbamoyl, 4-N,N-Dimethylcarbamoyl, 2-N,N-Diethylcarbamoyl, 3-N,N-Diethylcarbamoyl,4-N,N-Diethylcarbamoyl, 2-Amino, 3-Amino, 4-Amino, 2-N-Methylamino, 2-N-Methylamino, 4-N-Methylamino, 2-Dimethylamino, 3-Dimethylamino, 4-Dimethylamino, 2-N-Acetylamino, 3-N-Acetylamino, 4-N-Acetylamino, 2-Hydroxy, 3-Hydroxy, 4-Hydroxy, 2-Phenyl, 3-Phenyl, 4-Phenyl, 2-Sulfonylmethyl, 3-Sulfonylmethyl, 4-Sulfonylmethyl, 3-t-Butoxy, 4-t-Butoxy, 3-O-CH($CH_3$)$CO_2$Me, 4-O-CH($CH_3$)$CO_2$Me, 3-N-Piperidyl, 4-N-Piperidyl, 4-N-Pyrrolidinyl, 3-N-Pyrrolidinyl, 3-N-Pyrrolyl, 4-N-Pyrrolyl, 2,3-Difluor, 2,4-Difluor, 2,5-Difluor, 2,6-Difluor, 2,3,4-Trifluor, 2,3,5-Trifluor, 2,3,6-Trifluor, 2,4,5-Trifluor, 2,4,6-Trifluor, 2,3-Dichlor, 2,4-Dichlor, 2,5-Dichlor, 2,6-Dichlor, 2,3,4-Trichlor, 2,3,5-Trichlor, 2,3,6-Trichlor, 2,4,5-Trichlor, 2,4,6-Trichlor, 2,3-Dimethyl, 2,4-Dimethyl, 2,5-Dimethyl, 2,6-Dimethyl, 2,3,4-Trimethyl, 2,3,5-Trimethyl, 2,3,6-Trimethyl, 2,4,5-Trimethyl, 2,4,6-Trimethyl, 2-Fluor-6-Methyl, 2-Fluor-6-Ethyl, 2-Fluor-6-Chlor, 2-Fluor-6-Methoxy, 2-Fluor-6-N,N-Dimethylamino, 2-Fluor-6-Trifluormethyl, 2-Fluor-6-Carbomethoxy, 2-Fluor-6-Nitro, 2-Fluor-5-Methyl, 2-Fluor-5-Chlor, 2-Fluor-5-Methoxy, 2-Fluor-5-N,N-Dimethylamino, 2-Fluor-5-Trifluormethyl, 2-Fluor-5-Carbomethoxy, 2-Fluor-5-Nitro, 2-Fluor-5-Trifluormethoxy,2-Fluor-4-Methyl, 2-Fluor-4-Chlor, 2-Fluor-4-Methoxy, 2-Fluor-4-N,N-Dimethylamino, 2-Fluor-4-Trifluormethyl, 2-Fluor-4-Carbomethoxy, 2-Fluor-4-Nitro, 2-Fluor-4-Cyano, 2-Fluor-4-Sulfonylmethyl, 2-Fluor-3-Methyl, 2-Fluor-3-Chlor, 2-Fluor-3-Methoxy, 2-Fluor-3-N,N-Dimethylamino, 2-Fluor-3-Trifluormethyl, 2-Fluor-3-Carbomethoxy, 2-Fluor-3-Nitro, 2-Fluor-3-Trifluormethoxy, 2-Fluor-4-Chlor-5-Methoxy, 2-Chlor-6-Methyl, 2-Chlor-6-Ethyl, 2-Chlor-6-Fluor, 2-Chlor-6-Methoxy, 2-Chlor-6-Nitro, 2-Chlor-6-Dimethylamino, 2-Chlor-6-Trifluormethoxy, 2-Chlor-6-Carbomethoxy, 2-Chlor-5-Methyl, 2,4-Dichlor-5-Methyl, 2-Chlor-5-Fluor, 2,4-Dichlor-5-Fluor, 2-Chlor-5-Methoxy, 2-Chlor-5-Nitro, 2-Chlor-5-Dimethylamino, 2-Chlor-5-Trifluormethoxy, 2-Chlor-5-Carbomethoxy, 2-Chlor-5-trifluormethoxy, 2-Chlor-4-Methyl, 2-Chlor-4-Fluor, 2-Chlor-4,5-Difluor, 2-Chlor-4-Methoxy, 2-Chlor-4-Nitro, 2-Chlor-4-Dimethylamino, 2-Chlor-4-Trifluormethoxy, 2-Chlor-4-Carbomethoxy, 2-Chlor-4-Cyano, 2-Chlor-4-Sulfonylmethyl, 2-Chlor-3-Methyl, 2-Chlor-3-Fluor, 2-Chlor-3-Methoxy, 2-Chlor-3-Nitro, 2-Chlor-3-Dimethylamino, 2-Chlor-3-Trifluormethoxy, 2-Chlor-3-Carbomethoxy, 2-Chlor-3-Trifluormethoxy, 2-Methyl-6-Fluor, 2-Methyl-6-Chlor, 2-Methyl-6-Ethyl, 2-Methyl-6-Methoxy, 2-Methyl-6-Dimethylamino, 2-Methyl-6-Trifluormethyl, 2-Methyl-6-Carbomethoxy, 2-Methyl-6-Nitro, 2-Methyl-5-Fluor, 2-Methyl-5-Chlor, 2-Methyl-5-Methoxy, 2-Methyl-5-Dimethylamino, 2-Methyl-5-Trifluormethyl, 2-Methyl-5-Carbomethoxy, 2-Methyl-6-Nitro, 2-Methyl-5-Trifluormethoxy,

2-Methyl-4-Fluor, 2-Methyl-4-Chlor, 2-Methyl-4-Methoxy, 2-Methyl-4-Dimethylamino, 2-Methyl-4-Trifluormethyl, 2-Methyl-4-Carbomethoxy, 2-Methyl-4-Nitro, 2-Methyl-4-Cyano, 2-Methyl-4-Sulfonylmethyl, 2-Methyl-3-Fluor, 2-Methyl-3-Chlor, 2-Methyl-3-Methoxy, 2-Methyl-3-Dimethylamino, 2-Methyl-3-Trifluormethyl, 2-Methyl-3-Carbomethoxy, 2-Methyl-3-Nitro, 2-Methyl-3-Trifluormethoxy.

Wenn der Substituent $R^1$ die Bedeutung substituiertes 2-Thienyl hat, sind folgende Bedeutungen als besonders bevorzugt anzusehen:

2-Thienyl substituiert mit:

5-Methyl, 4-Methyl, 3-Methyl, 3,5-Dimethyl, 5-Ethyl, 3-Ethyl, 5-t-Butyl, 5-t-Butyl-4-amino, 5-Phenyl, 5-Phenyl-4-amino, 5-(2-Thienyl), 5-Chlor, 5-Brom, 5-Cyano, 5-Nitro;

Wenn der Substituent $R^1$ die Bedeutung substituiertes 3-Thienyl hat, sind folgende Bedeutungen als besonders bevorzugt anzusehen:

3-Thienyl substituiert mit:

5-Methyl, 2,5-Methyl, 5-Ethyl, 2-Methyl, 2-Ethyl, 5-Chlor, 5-Brom, 2,5-Dichlor, 2,5-Dibrom, 5-Nitro, 5-Phenyl;

Für den Substituenten $R^2$ sind folgenden Bedeutungen besonders bevorzugt:

$R^2$ = Wasserstoff, Methyl, Ethyl, n-Propyl, iso-Propyl, n-Butyl, s-Butyl, t-Butyl, Trifluormethyl, Cyclopropyl, Cyclopentyl, Cyclohexyl, Phenyl, Benzyl, Cyano, Carbomethoxy, Carboethoxy, Sulfonylmethyl, Sulfonylphenyl.

Für die Substituenten $R^3$-$R^6$ sind besonders bevorzugt:

$R^3$-$R^6$ = Wasserstoff, Phenyl, Thienyl, Methyl, Ethyl, i-Propyl, n-Propyl, Trifluormethyl, Benzyl, Nitro, Cyano,

Besonders bevorzugt sind die im folgenden genannten Verbindungen:

Verbindungen der Formel Ia

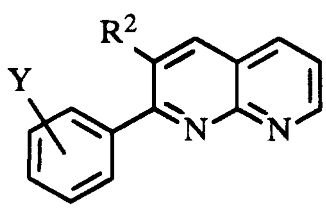

Ia

in der die Substituenten folgende Bedeutung haben:

$R^2$ = Wasserstoff und Y bedeutet jeweils:

2-Methyl, 3-Methyl, 4-Methyl, 2-Ethyl, 3-Ethyl, 4-Ethyl, 2-Propyl, 3-Propyl, 4-Propyl, 2-i-Propyl, 3-i-Propyl, 4-i-Propyl, 2-n-Butyl, 3-n-Butyl, 4-n-Butyl, 2-s-Butyl, 3-s-Butyl, 4-s-Butyl, 2-t-Butyl, 3-t-Butyl, 4-t-Butyl, 2-Benzyl, 3-Benzyl, 4-Benzyl, 2-cyclo-Propyl, 3-cyclo-Propyl, 4-cyclo-Propyl, 2-Trifluormethyl, 3-Trifluormethyl, 4-Trifluormethyl, 2-Methoxy, 3-Methoxy, 4-Methoxy, 2-Ethoxy, 3-Ethoxy, 4-Ethoxy, 2-Trifluormethoxy, 3-Trifluormethoxy, 4-Trifluormethoxy, 2-Thiomethyl, 3-Thiomethyl, 4-Thiomethyl, 2-Fluor, 3-Fluor, 4-Fluor, 2-Chlor, 3-Chlor, 4-Chlor, 2-Brom, 3-Brom, 4-Brom, 2-Nitro, 3-Nitro, 4-Nitro, 2-Cyano, 3-Cyano, 4-Cyano, 2-Carbomethoxy, 3-Carbomethoxy, 4-Carbomethoxy, 2-Carboethoxy, 3-Carboethoxy, 4-Carboethoxy, 2-Carbamoyl, 3-Carbamoyl, 4-Carbamoyl, 2-N-Methylcarbamoyl, 3-N-Methylcarbamoyl, 4-N-Methylcarbamoyl, 2-N,N-Dimethylcarbamoyl, 3-N,N-Dimethylcarbamoyl, 4-N,N-Dimethylcarbamoyl, 2-N,N-Diethylcarbamoyl, 3-N,N-Diethylcarbamoyl, 4-N,N-Diethylcarbamoyl, 2-Amino, 3-Amino, 4-Amino, 2-N-Methylamino, 2-N-Methylamino, 4-N-Methylamino, 2-Dimethylamino, 3-Dimethylamino, 4-Dimethylamino, 2-N-Acetylamino, 3-N-Acetylamino, 4-N-Acetylamino, 2-Hydroxy, 3-Hydroxy, 4-Hydroxy, 2-Phenyl, 3-Phenyl, 4-Phenyl, 2-Sulfonylmethyl, 3-Sulfonylmethyl, 4-Sulfonylmethyl, 3-t-Butoxy, 4-t-Butoxy, 3-O-CH($CH_3$)$CO_2$Me, 4-O-CH($CH_3$)$CO_2$Me, 3-N-Piperidyl, 4-N-Piperidyl, 4-N-Pyrrolidinyl, 3-Pyrrolidinyl, 3-N-Pyrrolyl, 4-N-Pyrrolyl, 2,3-Difluor, 2,4-Difluor, 2,5-Difluor, 2,6-Difluor, 2,3,4-Trifluor, 2,3,5-Trifluor, 2,3,6-Trifluor, 2,4,5-Trifluor, 2,4,6-Trifluor, 2,3-Dichlor, 2,4-Dichlor, 2,5-Dichlor, 2,6-Dichlor, 2,3,4-Trichlor, 2,3,5-Trichlor, 2,3,6-Trichlor, 2,4,5-Trichlor, 2,4,6-Trichlor, 2,3-Dimethyl, 2,4-Dimethyl, 2,5-Dimethyl, 2,6-Dimethyl, 2,3,4-Trimethyl, 2,3,5-Trimethyl, 2,3,6-Trimethyl, 2,4,5-Trimethyl, 2,4,6-Trimethyl, 2-Fluor-6-Methyl, 2-Fluor-6-Ethyl, 2-Fluor-6-Chlor, 2-Fluor-6-Methoxy, 2-Fluor-6-N,N-Dimethylamino, 2-Fluor-6-Trifluormethyl, 2-Fluor-6-Carbomethoxy, 2-Fluor-6-Nitro,2-Fluor-5-Methyl, 2-Fluor-5-Chlor, 2-Fluor-5-Methoxy, 2-Fluor-5-N,N-Dimethylamino, 2-Fluor-5-Trifluormethyl, 2-Fluor-5-Carbomethoxy, 2-Fluor-5-Nitro, 2-Fluor-5-Trifluormethoxy,2-Fluor-4-Methyl, 2-Fluor-4-Chlor, 2-Fluor-4-Methoxy, 2-Fluor-4-N,N-Dimethylamino, 2-Fluor-4-Trifluormethyl, 2-Fluor-4-Carbomethoxy, 2-Fluor-4-Nitro, 2-Fluor-4-Cyano, 2-Fluor-4-Sulfonylmethyl,2-Fluor-3-Methyl, 2-Fluor-3-Chlor, 2-Fluor-3-Methoxy, 2-Fluor-3-N,N-Dimethylamino, 2-Fluor-3-Trifluormethyl, 2-Fluor-3-Carbomethoxy, 2-Fluor-3-Nitro, 2-Fluor-3-Trifluormethoxy, 2-Fluor-4-Chlor-5-Methoxy, 2-Chlor-6-Methyl, 2-Chlor-6-Ethyl, 2-Chlor-6-Fluor, 2-Chlor-6-Methoxy, 2-Chlor-6-Nitro, 2-Chlor-6-Dimethylamino, 2-Chlor-6-Trifluormethoxy, 2-Chlor-6-Carbomethoxy, 2-Chlor-5-Methyl, 2,4-Dichlor-5-Methyl, 2-Chlor-5-Fluor, 2,4-Dichlor-5-Fluor, 2-Chlor-5-Methoxy, 2-Chlor-5-Nitro, 2-Chlor-5-Dimethylamino, 2-Chlor-5-Trifluormethoxy, 2-Chlor-5-Carbomethoxy, 2-Chlor-5-trifluormethoxy, 2-Chlor-4-Methyl, 2-Chlor-4-Fluor, 2-Chlor-4,5-Difluor, 2-Chlor-4-Methoxy, 2-Chlor-4-Nitro, 2-Chlor-4-Dimethylamino, 2-Chlor-4-Trifluormethoxy, 2-Chlor-4-Carbomethoxy, 2-Chlor-4-Cyano, 2-Chlor-4-Sulfonylmethyl,2-Chlor-3-Methyl, 2-Chlor-3-Fluor, 2-Chlor-3-Methoxy, 2-Chlor-3-Nitro, 2-Chlor-3-Dimethylamino, 2-Chlor-3-Trifluormethoxy, 2-Chlor-3-Carbomethoxy, 2-Chlor-3-Trifluormethoxy, 2-Methyl-6-Fluor, 2-Methyl-6-Chlor, 2-Methyl-6-Ethyl, 2-Methyl-6-Methoxy, 2-Methyl-6-Dimethylamino, 2-Methyl-6-Trifluormethyl, 2-Methyl-6-Carbomethoxy, 2-Methyl-6-Nitro, 2-Methyl-5-Fluor, 2-Methyl-5-Chlor, 2-Methyl-5-Methoxy, 2-Methyl-5-Dimethylamino, 2-Methyl-5-Trifluormethyl, 2-Methyl-5-Carbomethoxy, 2-Methyl-6-Nitro, 2-Methyl-5-Trifluormethoxy, 2-Methyl-4-Fluor, 2-Methyl-4-Chlor, 2-Methyl-4-Methoxy, 2-Methyl-4-Dimethylamino, 2-Methyl-4-Trifluormethyl, 2-Methyl-4-Carbomethoxy, 2-Methyl-4-Nitro, 2-Methyl-4-Cyano, 2-Methyl-4-Sulfonylmethyl,2-Methyl-3-Fluor, 2-Methyl-3-Chlor, 2-Methyl-3-Methoxy, 2-Methyl-3-Dimethylamino, 2-Methyl-3-Trifluormethyl, 2-Methyl-3-Carbomethoxy, 2-Methyl-3-Nitro, 2-Methyl-3-Trifluormethoxy.

Verbindungen der Formel Ia mit $R^2$ = Methyl und Y bedeutet jeweils:

2-Methyl, 3-Methyl, 4-Methyl, 2-Ethyl, 3-Ethyl, 4-Ethyl, 2-Propyl, 3-Propyl, 4-Propyl, 2-i-Propyl, 3-i-Propyl, 4-i-Propyl, 2-n-Butyl, 3-n-Butyl, 4-n-Butyl, 2-s-Butyl, 3-s-Butyl, 4-s-Butyl, 2-t-Butyl, 3-t-Butyl, 4-t-Butyl, 2-Benzyl, 3-Benzyl, 4-Benzyl, 2-cyclo-Propyl, 3-cyclo-Propyl, 4-cyclo-Propyl, 2-Trifluormethyl, 3-Trifluormethyl, 4-Trifluormethyl, 2-Methoxy, 3-Methoxy, 4-Methoxy, 2-Ethoxy, 3-Ethoxy, 4-Ethoxy, 2-Trifluormethoxy, 3-Trifluormethoxy, 4-Trifluormethoxy, 2-Thiomethyl, 3-Thiomethyl, 4-Thiomethyl, 2-Fluor, 3-Fluor, 4-Fluor, 2-Chlor, 3-Chlor, 4-Chlor, 2-Brom, 3-Brom, 4-Brom, 2-Nitro, 3-Nitro, 4-Nitro, 2-Cyano, 3-Cyano, 4-Cyano, 2-Carbomethoxy, 3-Carbomethoxy, 4-Carbomethoxy, 2-Carboethoxy, 3-Carboethoxy, 4-Carboethoxy, 2-Carbamoyl, 3-Carbamoyl, 4-Carbamoyl, 2-N-Methylcarbamoyl, 3-N-Methylcarbamoyl, 4-N-Methylcarbamoyl, 2-N,N-Dimethylcarbamoyl, 3-N,N-Dimethylcarbamoyl, 4-N,N-Dimethylcarbamoyl, 2-N,N-Diethylcarbamoyl, 3-N,N-Diethylcarbamoyl, 4-N,N-Diethylcarbamoyl, 2-Amino, 3-Amino, 4-Amino, 2-N-Methylamino, 2-N-Methylamino, 4-N-Methylamino, 2-Dimethylamino, 3-Dimethylamino, 4-Dimethylamino, 2-N-Acetylamino, 3-N-Acetylamino, 4-N-Acetylamino, 2-Hydroxy, 3-Hydroxy, 4-Hydroxy, 2-Phenyl, 3-Phenyl, 4-Phenyl, 2-Sulfonylmethyl, 3-Sulfonylmethyl, 4-Sulfonylmethyl, 3-t-Butoxy, 4-t-Butoxy, 3-O-$CH(CH_3)CO_2Me$, 4-O-$CH(CH_3)CO_2Me$, 3-N-Piperidyl, 4-N-Piperidyl, 4-N-Pyrrolidinyl, 3-Pyrrolidinyl, 3-N-Pyrrolyl, 4-N-Pyrrolyl, 2,3-Difluor, 2,4-Difluor, 2,5-Difluor, 2,6-Difluor, 2,3,4-Trifluor, 2,3,5-Trifluor, 2,3,6-Trifluor, 2,4,5-Trifluor, 2,4,6-Trifluor, 2,3-Dichlor, 2,4-Dichlor, 2,5-Dichlor, 2,6-Dichlor, 2,3,4-Trichlor, 2,3,5-Trichlor, 2,3,6-Trichlor, 2,4,5-Trichlor, 2,4,6-Trichlor, 2,3-Dimethyl, 2,4-Dimethyl, 2,5-Dimethyl, 2,6-Dimethyl, 2,3,4-Trimethyl, 2,3,5-Trimethyl, 2,3,6-Trimethyl, 2,4,5-Trimethyl, 2,4,6-Trimethyl, 2-Fluor-6-Methyl, 2-Fluor-6-Ethyl, 2-Fluor-6-Chlor, 2-Fluor-6-Methoxy, 2-Fluor-6-N,N-Dimethylamino, 2-Fluor-6-Trifluormethyl, 2-Fluor-6-Carbomethoxy, 2-Fluor-6-Nitro,2-Fluor-5-Methyl, 2-Fluor-5-Chlor, 2-Fluor-5-Methoxy, 2-Fluor-5-N,N-Dimethylamino, 2-Fluor-5-Trifluormethyl, 2-Fluor-5-Carbomethoxy, 2-Fluor-5-Nitro, 2-Fluor-5-Trifluormethoxy, 2-Fluor-4-Methyl, 2-Fluor-4-Chlor, 2-Fluor-4-Methoxy, 2-Fluor-4-N,N-Dimethylamino, 2-Fluor-4-Trifluormethyl, 2-Fluor-4-Carbomethoxy, 2-Fluor-4-Nitro, 2-Fluor-4-Cyano, 2-Fluor-4-Sulfonylmethyl,2-Fluor-3-Methyl, 2-Fluor-3-Chlor, 2-Fluor-3-Methoxy, 2-Fluor-3-N,N-Dimethylamino, 2-Fluor-3-Trifluormethyl, 2-Fluor-3-Carbomethoxy, 2-Fluor-3-Nitro, 2-Fluor-3-Trifluormethoxy, 2-Fluor-4-Chlor-5-Methoxy, 2-Chlor-6-Methyl, 2-Chlor-6-Ethyl, 2-Chlor-6-Fluor, 2-Chlor-6-Methoxy, 2-Chlor-6-Nitro, 2-Chlor-6-Dimethylamino, 2-Chlor-6-Trifluormethoxy, 2-Chlor-6-Carbomethoxy,2-Chlor-5-Methyl, 2,4-Dichlor-5-Methyl, 2-Chlor-5-Fluor, 2,4-Dichlor-5-Fluor, 2-Chlor-5-Methoxy, 2-Chlor-5-Nitro, 2-Chlor-5-Dimethylamino, 2-Chlor-5-Trifluormethoxy, 2-Chlor-5-Carbomethoxy, 2-Chlor-5-trifluormethoxy, 2-Chlor-4-Methyl, 2-Chlor-4-Fluor, 2-Chlor-4,5-Difluor, 2-Chlor-4-Methoxy, 2-Chlor-4-Nitro, 2-Chlor-4-Dimethylamino, 2-Chlor-4-Trifluormethoxy, 2-Chlor-4-Carbomethoxy, 2-Chlor-4-Cyano, 2-Chlor-4-Sulfonylmethyl,2-Chlor-3-Methyl, 2-Chlor-3-Fluor, 2-Chlor-3-Methoxy, 2-Chlor-3-Nitro, 2-Chlor-3-Dimethylamino, 2-Chlor-3-Trifluormethoxy, 2-Chlor-3-Carbomethoxy, 2-Chlor-3-Trifluormethoxy, 2-Methyl-6-Fluor, 2-Methyl-6-Chlor, 2-Methyl-6-Ethyl, 2-Methyl-6-Methoxy, 2-Methyl-6-Dimethylamino, 2-Methyl-6-Trifluormethyl, 2-Methyl-6-Carbomethoxy, 2-Methyl-6-Nitro, 2-Methyl-5-Fluor, 2-Methyl-5-Chlor, 2-Methyl-5-Methoxy, 2-Methyl-5-Dimethylamino, 2-Methyl-5-Trifluormethyl, 2-Methyl-5-Carbomethoxy, 2-Methyl-6-Nitro, 2-Methyl-5-Trifluormethoxy, 2-Methyl-4-Fluor, 2-Methyl-4-Chlor, 2-Methyl-4-Methoxy, 2-Methyl-4-Dimethylamino, 2-Methyl-4-Trifluormethyl, 2-Methyl-4-Carbomethoxy, 2-Methyl-4-Nitro, 2-Methyl-4-Cyano, 2-Methyl-4-Sulfonylmethyl,2-Methyl-3-Fluor, 2-Methyl-3-Chlor, 2-Methyl-3-Methoxy, 2-Methyl-3-Dimethylamino, 2-Methyl-3-Trifluormethyl, 2-Methyl-3-Carbomethoxy, 2-Methyl-3-Nitro, 2-Methyl-3-Trifluormethoxy,

Verbindungen der Formel Ia mit $R^2$ = Ethyl und Y bedeutet jeweils:

2-Methyl, 3-Methyl, 4-Methyl, 2-Ethyl, 3-Ethyl, 4-Ethyl, 2-Propyl, 3-Propyl, 4-Propyl, 2-i-Propyl, 3-i-Propyl, 4-i-Propyl, 2-n-Butyl, 3-n-Butyl, 4-n-Butyl, 2-s-Butyl, 3-s-Butyl, 4-s-Butyl, 2-t-Butyl, 3-t-Butyl, 4-t-Butyl, 2-Benzyl, 3-Benzyl, 4-Benzyl, 2-cyclo-Propyl, 3-cyclo-Propyl, 4-cyclo-Propyl, 2-Trifluormethyl, 3-Trifluormethyl, 4-Trifluormethyl, 2-Methoxy, 3-Methoxy, 4-Methoxy, 2-Ethoxy, 3-Ethoxy, 4-Ethoxy, 2-Trifluormethoxy, 3-Trifluormethoxy, 4-Trifluormethoxy, 2-Thiomethyl, 3-Thiomethyl, 4-Thiomethyl, 2-Fluor, 3-Fluor, 4-Fluor, 2-Chlor, 3-Chlor, 4-Chlor, 2-Brom, 3-Brom, 4-Brom, 2-Nitro, 3-Nitro, 4-Nitro, 2-Cyano, 3-Cyano, 4-Cyano, 2-Carbomethoxy, 3-Carbomethoxy, 4-Carbomethoxy, 2-Carboethoxy, 3-Carboethoxy, 4-Carboethoxy, 2-Carbamoyl, 3-Carbamoyl, 4-Carbamoyl, 2-N-Methylcarbamoyl, 3-N-Methylcarbarmoyl, 4-N-Methylcarbamoyl, 2-N,N-Dimethylcarbamoyl, 3-N,N-Dimethylcarbamoyl, 4-N,N-Dimethylcarbamoyl, 2-N,N-Diethylcarbamoyl, 3-N,N-Diethylcarbamoyl, 4-N,N-Diethylcarbamoyl, 2-Amino, 3-Amino, 4-Amino, 2-N-Methylamino, 2-N-Methylamino, 4-N-Methylamino, 2-Dimethylamino, 3-Dimethylamino, 4-Dimethylamino, 2-N-Acetylamino, 3-N-Acetylamino, 4-N-Acetylamino, 2-Hydroxy, 3-Hydroxy, 4-Hydroxy, 2-Phenyl, 3-Phenyl, 4-Phenyl, 2-Sulfonylmethyl, 3-Sulfonylmethyl, 4-Sulfonylmethyl, 3-t-Butoxy, 4-t-Butoxy, 3-O-$CH(CH_3)CO_2Me$, 4-O-$CH(CH_3)CO_2Me$, 3-N-Piperidyl, 4-N-Piperidyl, 4-N-Pyrrolidinyl, 3-Pyrrolidinyl, 3-N-Pyrrolyl, 4-N-Pyrrolyl, 2,3-Difluor, 2,4-Difluor, 2,5-Difluor, 2,6-Difluor, 2,3,4-Trifluor, 2,3,5-Trifluor, 2,3,6-Trifluor, 2,4,5-Trifluor, 2,4,6-Trifluor, 2,3-Dichlor, 2,4-Dichlor, 2,5-Dichlor, 2,6-Dichlor, 2,3,4-Trichlor, 2,3,5-Trichlor, 2,3,6-Trichlor, 2,4,5-Trichlor, 2,4,6-Trichlor, 2,3-Dimethyl, 2,4-Dimethyl, 2,5-Dimethyl, 2,6-Dimethyl, 2,3,4-Trimethyl, 2,3,5-Trimethyl, 2,3,6-Trimethyl, 2,4,5-Trimethyl, 2,4,6-Trimethyl, 2-Fluor-6-Methyl, 2-Fluor-6-Ethyl, 2-Fluor-6-Chlor, 2-Fluor-6-Methoxy, 2-Fluor-6-N,N-Dimethylamino, 2-Fluor-6-Trifluormethyl, 2-Fluor-6-Carbomethoxy, 2-Fluor-6-Nitro,2-Fluor-5-Methyl, 2-Fluor-5-Chlor, 2-Fluor-5-Methoxy, 2-Fluor-5-N,N-Dimethylamino, 2-Fluor-5-Trifluormethyl, 2-Fluor-5-Carbomethoxy, 2-Fluor-5-Nitro, 2-Fluor-5-Trifluormethoxy,2-Fluor-4-Methyl, 2-Fluor-4-Chlor, 2-Fluor-4-Methoxy, 2-Fluor-4-N,N-Dimethylamino, 2-Fluor-4-Trifluormethyl, 2-Fluor-4-Carbomethoxy, 2-Fluor-4-Nitro, 2-Fluor-4-Cyano, 2-Fluor-4-Sulfonylmethyl,2-Fluor-3-Methyl, 2-Fluor-3-Chlor, 2-Fluor-3-Methoxy, 2-Fluor-3-N,N-Dimethylamino, 2-Fluor-3-Trifluormethyl, 2-Fluor-3-Carbomethoxy, 2-Fluor-3-Nitro, 2-Fluor-3-Trifluormethoxy, 2-Fluor-4-Chlor-5-Methoxy, 2-Chlor-6-Methyl, 2-Chlor-6-Ethyl, 2-Chlor-6-Fluor, 2-Chlor-6-Methoxy, 2-Chlor-6-Nitro, 2-Chlor-6-Dimethylamino, 2-Chlor-6-Trifluormethoxy, 2-Chlor-6-Carbomethoxy,2-Chlor-5-Methyl, 2,4-Dichlor-5-Methyl, 2-Chlor-5-Fluor, 2,4-Dichlor-5-Fluor, 2-Chlor-5-Methoxy, 2-Chlor-5-Nitro, 2-Chlor-5-Dimethylamino, 2-Chlor-5-Trifluormethoxy, 2-Chlor-5-Carbomethoxy, 2-Chlor-5-trifluormethoxy, 2-Chlor-4-Methyl, 2-Chlor-4-Fluor, 2-Chlor-4,5-Difluor, 2-Chlor-4-Methoxy, 2-Chlor-4-Nitro, 2-Chlor-4-Dimethylamino, 2-Chlor-4-Trifluormethoxy, 2-Chlor-4-Carbomethoxy, 2-Chlor-4-Cyano, 2-Chlor-4-Sulfonylmethyl,2-Chlor-3-Methyl, 2-Chlor-3-Fluor, 2-Chlor-3-Methoxy, 2-Chlor-3-Nitro, 2-Chlor-3-Dimethylamino, 2-Chlor-3-Trifluormethoxy, 2-Chlor-3-Carbomethoxy, 2-Chlor-3-Trifluormethoxy, 2-Methyl-6-Fluor, 2-Methyl-6-Chlor, 2-Methyl-6-Ethyl, 2-Methyl-6-Methoxy, 2-Methyl-6-Dimethylamino, 2-Methyl-6-Trifluormethyl, 2-Methyl-6-Carbomethoxy, 2-Methyl-6-Nitro, 2-Methyl-5-Fluor, 2-Methyl-5-Chlor, 2-Methyl-5-Methoxy, 2-Methyl-5-Dimethylamino, 2-Methyl-5-Trifluormethyl, 2-Methyl-5-Carbomethoxy, 2-Methyl-6-Nitro, 2-Methyl-5-Trifluormethoxy, 2-Methyl-4-Fluor, 2-Methyl-4-Chlor, 2-Methyl-4-Methoxy, 2-Methyl-4-Dimethylamino, 2-Methyl-4-Trifluormethyl, 2-Methyl-4-Carbomethoxy, 2-Methyl-4-Nitro, 2-Methyl-4-Cyano, 2-Methyl-4-Sulfonylmethyl,2-Methyl-3-Fluor, 2-Methyl-3-Chlor, 2-Methyl-3-Methoxy, 2-Methyl-3-Dimethylamino, 2-Methyl-3-Trifluormethyl, 2-Methyl-3-Carbomethoxy, 2-Methyl-3-Nitro, 2-Methyl-3-Trifluormethoxy,

Verbindungen der Formel Ia mit $R^2$ = iso-Propyl und Y bedeutet jeweils:

2-Methyl, 3-Methyl, 4-Methyl, 2-Ethyl, 3-Ethyl, 4-Ethyl, 2-Propyl, 3-Propyl, 4-Propyl, 2-i-Propyl, 3-i-Propyl, 4-i-Propyl, 2-n-Butyl, 3-n-Butyl, 4-n-Butyl, 2-s-Butyl, 3-s-Butyl, 4-s-Butyl, 2-t-Butyl, 3-t-Butyl, 4-t-Butyl, 2-Benzyl, 3-Benzyl, 4-Benzyl, 2-cyclo-Propyl, 3-cyclo-Propyl, 4-cyclo-Propyl, 2-Trifluormethyl, 3-Trifluormethyl, 4-Trifluormethyl, 2-Methoxy, 3-Methoxy, 4-Methoxy, 2-Ethoxy, 3-Ethoxy, 4-Ethoxy, 2-Trifluormethoxy, 3-Trifluormethoxy, 4-Trifluormethoxy, 2-Thiomethyl, 3-Thiomethyl, 4-Thiomethyl, 2-Fluor, 3-Fluor, 4-Fluor, 2-Chlor, 3-Chlor, 4-Chlor, 2-Brom, 3-Brom, 4-Brom, 2-Nitro, 3-Nitro, 4-Nitro, 2-Cyano, 3-Cyano, 4-Cyano, 2-Carbomethoxy, 3-Carbomethoxy, 4-Carbomethoxy, 2-Carboethoxy, 3-Carboethoxy, 4-Carboethoxy, 2-Carbamoyl, 3-Carbamoyl, 4-Carbamoyl, 2-N-Methylcarbamoyl, 3-N-Methylcarbamoyl, 4-N-Methylcarbamoyl, 2-N,N-Dimethylcarbamoyl, 3-N,N-Dimethylcarbamoyl, 4-N,N-Dimethylcarbamoyl, 2-N,N-Diethylcarbamoyl, 3-N,N-Diethylcarbamoyl, 4-N,N-Diethylcarbamoyl, 2-Amino, 3-Amino, 4-Amino, 2-N-Methylamino, 2-N-Methylamino, 4-N-Methylamino, 2-Dimethylamino, 3-Dimethylamino, 4-Dimethylamino, 2-N-Acetylamino, 3-N-Acetylamino, 4-N-Acetylamino, 2-Hydroxy, 3-Hydroxy, 4-Hydroxy, 2-Phenyl, 3-Phenyl, 4-Phenyl, 2-Sulfonylmethyl, 3-Sulfonylmethyl, 4-Sulfonylmethyl, 3-t-Butoxy, 4-t-Butoxy, 3-O-$CH(CH_3)CO_2Me$, 4-O-$CH(CH_3)CO_2Me$, 3-N-Piperidyl, 4-N-Piperidyl, 4-N-Pyrrolidinyl, 3-Pyrrolidinyl, 3-N-Pyrrolyl, 4-N-Pyrrolyl, 2,3-Difluor, 2,4-Difluor, 2,5-Difluor, 2,6-Difluor, 2,3,4-Trifluor, 2,3,5-Trifluor, 2,3,6-Trifluor, 2,4,5-Trlfluor, 2,4,6-Trifluor, 2,3-Dichlor, 2,4-Dichlor, 2,5-Dichlor, 2,6-Dichlor, 2,3,4-Trichlor, 2,3,5-Trichlor, 2,3,6-Trichlor, 2,4,5-Trichlor, 2,4,6-Trichlor, 2,3-Dimethyl, 2,4-Dimethyl, 2,5-Dimethyl, 2,6-Dimethyl, 2,3,4-Trimethyl, 2,3,5-Trimethyl, 2,3,6-Trimethyl, 2,4,5-Trimethyl, 2,4,6-Trimethyl, 2-Fluor-6-Methyl, 2-Fluor-6-Ethyl, 2-Fluor-6-Chlor, 2-Fluor-6-Methoxy, 2-Fluor-6-N,N-Dimethylamino, 2-Fluor-6-Trifluormethyl, 2-Fluor-6-Carbomethoxy, 2-Fluor-6-Nitro, 2-Fluor-5-Methyl, 2-Fluor-5-Chlor, 2-Fluor-5-Methoxy, 2-Fluor-5-N,N-Dimethylamino, 2-Fluor-5-Trifluormethyl, 2-Fluor-5-Carbomethoxy, 2-Fluor-5-Nitro, 2-Fluor-5-Trifluormethoxy,2-Fluor-4-Methyl, 2-Fluor-4-Chlor,

2-Fluor-4-Methoxy, 2-Fluor-4-N,N-Dimethylamino, 2-Fluor-4-Trifluormethyl, 2-Fluor-4-Carbomethoxy, 2-Fluor-4-Nitro, 2-Fluor-4-Cyano, 2-Fluor-4-Sulfonylmethyl, 2-Fluor-3-Methyl, 2-Fluor-3-Chlor, 2-Fluor-3-Methoxy, 2-Fluor-3-N,N-Dimethylamino, 2-Fluor-3-Trifluormethyl, 2-Fluor-3-Carbomethoxy, 2-Fluor-3-Nitro, 2-Fluor-3-Trifluormethoxy, 2-Fluor-4-Chlor-5-Methoxy, 2-Chlor-6-Methyl, 2-Chlor-6-Ethyl, 2-Chlor-6-Fluor, 2-Chlor-6-Methoxy, 2-Chlor-6-Nitro, 2-Chlor-6-Dimethylamino, 2-Chlor-6-Trifluormethoxy, 2-Chlor-6-Carbomethoxy,2-Chlor-5-Methyl,2,4-Dichlor-5-Methyl, 2-Chlor-5-Fluor, 2,4-Dichlor-5-Fluor, 2-Chlor-5-Methoxy, 2-Chlor-5-Nitro, 2-Chlor-5-Dimethylamino, 2-Chlor-5-Trifluormethoxy, 2-Chlor-5-Carbomethoxy, 2-Chlor-5-trifluormethoxy, 2-Chlor-4-Methyl, 2-Chlor-4-Fluor, 2-Chlor-4,5-Difluor, 2-Chlor-4-Methoxy, 2-Chlor-4-Nitro, 2-Chlor-4-Dimethylamino, 2-Chlor-4-Trifluormethoxy, 2-Chlor-4-Carbomethoxy, 2-Chlor-4-Cyano, 2-Chlor-4-Sulfonylmethyl,2-Chlor-3-Methyl, 2-Chlor-3-Fluor, 2-Chlor-3-Methoxy, 2-Chlor-3-Nitro, 2-Chlor-3-Dimethylamino, 2-Chlor-3-Trifluormethoxy, 2-Chlor-3-Carbomethoxy, 2-Chlor-3-Trifluormethoxy, 2-Methyl-6-Fluor, 2-Methyl-6-Chlor, 2-Methyl-6-Ethyl, 2-Methyl-6-Methoxy, 2-Methyl-6-Dimethylamino, 2-Methyl-6-Trifluormethyl, 2-Methyl-6-Carbomethoxy, 2-Methyl-6-Nitro, 2-Methyl-5-Fluor, 2-Methyl-5-Chlor, 2-Methyl-5-Methoxy, 2-Methyl-5-Dimethylamino, 2-Methyl-5-Trifluormethyl, 2-Methyl-5-Carbomethoxy, 2-Methyl-6-Nitro, 2-Methyl-5-Trifluormethoxy, 2-Methyl-4-Fluor, 2-Methyl-4-Chlor, 2-Methyl-4-Methoxy, 2-Methyl-4-Dimethylamino, 2-Methyl-4-Trifluormethyl, 2-Methyl-4-Carbomethoxy, 2-Methyl-4-Nitro, 2-Methyl-4-Cyano, 2-Methyl-4-Sulfonylmethyl,2-Methyl-3-Fluor, 2-Methyl-3-Chlor, 2-Methyl-3-Methoxy, 2-Methyl-3-Dimethylamino, 2-Methyl-3-Trifluormethyl, 2-Methyl-3-Carbomethoxy, 2-Methyl-3-Nitro, 2-Methyl-3-Trifluormethoxy.

Verbindungen der Formel Ia mit $R^2$ = Ethyl und Y bedeutet jeweils:

2-Methyl, 3-Methyl, 4-Methyl, 2-Ethyl, 3-Ethyl, 4-Ethyl, 2-Propyl, 3-Propyl, 4-Propyl, 2-i-Propyl, 3-i-Propyl, 4-i-Propyl, 2-n-Butyl, 3-n-Butyl, 4-n-Butyl, 2-s-Butyl, 3-s-Butyl, 4-s-Butyl, 2-t-Butyl, 3-t-Butyl, 4-t-Butyl, 2-Benzyl, 3-Benzyl, 4-Benzyl, 2-cyclo-Propyl, 3-cyclo-Propyl, 4-cyclo-Propyl, 2-Trifluormethyl, 3-Trifluormethyl, 4-Trifluormethyl,2-Methoxy, 3-Methoxy, 4-Methoxy, 2-Ethoxy, 3-Ethoxy, 4-Ethoxy, 2-Trifluormethoxy, 3-Trifluormethoxy, 4-Trifluormethoxy, 2-Thiomethyl, 3-Thiomethyl, 4-Thiomethyl, 2-Fluor, 3-Fluor, 4-Fluor,2-Chlor, 3-Chlor, 4-Chlor, 2-Brom, 3-Brom, 4-Brom, 2-Nitro, 3-Nitro, 4-Nitro, 2-Cyano, 3-Cyano, 4-Cyano, 2-Carbomethoxy, 3-Carbomethoxy, 4-Carbomethoxy, 2-Carboethoxy, 3-Carboethoxy, 4-Carboethoxy, 2-Carbamoyl, 3-Carbamoyl, 4-Carbamoyl, 2-N-Methylcarbamoyl, 3-N-Methylcarbamoyl, 4-N-Methylcarbamoyl, 2-N,N-Dimethylcarbamoyl, 3-N,N-Dimethylcarbamoyl, 4-N,N-Dimethylcarbamoyl, 2-N,N-Diethylcarbamoyl, 3-N,N-Diethylcarbamoyl, 4-N,N-Diethylcarbamoyl, 2-Amino, 3-Amino, 4-Amino, 2-N-Methylamino, 2-N-Methylamino, 4-N-Methylamino, 2-Dimethylamino, 3-Dimethylamino, 4-Dimethylamino, 2-N-Acetylamino, 3-N-Acetylamino, 4-N-Acetylamino, 2-Hydroxy, 3-Hydroxy, 4-Hydroxy, 2-Phenyl, 3-Phenyl, 4-Phenyl, 2-Sulfonylmethyl, 3-Sulfonylmethyl, 4-Sulfonylmethyl, 3-t-Butoxy, 4-t-Butoxy, 3-O-$CH(CH_3)CO_2$Me, 4-O-$CH(CH_3)CO_2$Me, 3-N-Piperidyl, 4-N-Piperidyl, 4-N-Pyrrolidinyl, 3-Pyrrolidinyl, 3-N-Prrolyl, 4-N-Pyrrolyl, 2,3-Difluor, 2,4-Difluor, 2,5-Difluor, 2,6-Difluor, 2,3,4-Trifluor, 2,3,5-Trifluor, 2,3,6-Trifluor, 2,4,5-Trifluor, 2,4,6-Trifluor, 2,3-Dichlor, 2,4-Dichlor, 2,5-Dichlor, 2,6-Dichlor, 2,3,4-Trichlor, 2,3,5-Trichlor, 2,3,6-Trichlor, 2,4,5-Trichlor, 2,4,6-Trichlor, 2,3-Dimethyl, 2,4-Dimethyl, 2,5-Dimethyl, 2,6-Dimethyl, 2,3,4-Trimethyl, 2,3,5-Trimethyl, 2,3,6-Trimethyl, 2,4,5-Trimethyl, 2,4,6-Trimethyl, 2-Fluor-6-Methyl, 2-Fluor-6-Ethyl, 2-Fluor-6-Chlor, 2-Fluor-6-Methoxy, 2-Fluor-6-N,N-Dimethylamino, 2-Fluor-6-Trifluormethyl, 2-Fluor-6-Carbomethoxy, 2-Fluor-6-Nitro,2-Fluor-5-Methyl, 2-Fluor-5-Chlor, 2-Fluor-5-Methoxy, 2-Fluor-5-N,N-Dimethylamino, 2-Fluor-5-Trifluormethyl, 2-Fluor-5-Carbomethoxy, 2-Fluor-5-Nitro, 2-Fluor-5-Trifluormethoxy,2-Fluor-4-Methyl, 2-Fluor-4-Chlor, 2-Fluor-4-Methoxy, 2-Fluor-4-N,N-Dimethylamino, 2-Fluor-4-Trifluormethyl, 2-Fluor-4-Carbomethoxy, 2-Fluor-4-Nitro, 2-Fluor-4-Cyano, 2-Fluor-4-Sulfonylmethyl,2-Fluor-3-Methyl, 2-Fluor-3-Chlor, 2-Fluor-3-Methoxy, 2-Fluor-3-N,N-Dimethylamino, 2-Fluor-3-Trifluormethyl, 2-Fluor-3-Carbomethoxy, 2-Fluor-3-Nitro, 2-Fluor-3-Trifluormethoxy, 2-Fluor-4-Chlor-5-Methoxy, 2-Chlor-6-Methyl, 2-Chlor-6-Ethyl, 2-Chlor-6-Fluor, 2-Chlor-6-Methoxy, 2-Chlor-6-Nitro, 2-Chlor-6-Dimethylamino, 2-Chlor-6-Trifluormethoxy, 2-Chlor-6-Carbomethoxy,2-Chlor-5-Methyl, 2,4-Dichlor-5-Methyl, 2-Chlor-5-Fluor, 2,4-Dichlor-5-Fluor, 2-Chlor-5-Methoxy, 2-Chlor-5-Nitro, 2-Chlor-5-Dimethylamino, 2-Chlor-5-Trifluormethoxy, 2-Chlor-5-Carbomethoxy, 2-Chlor-5-trifluormethoxy, 2-Chlor-4-Methyl, 2-Chlor-4-Fluor, 2-Chlor-4,5-Difluor, 2-Chlor-4-Methoxy, 2-Chlor-4-Nitro, 2-Chlor-4-Dimethylamino, 2-Chlor-4-Trifluormethoxy, 2-Chlor-4-Carbomethoxy, 2-Chlor-4-Cyano, 2-Chlor-4-Sulfonylmethyl,2-Chlor-3-Methyl, 2-Chlor-3-Fluor, 2-Chlor-3-Methoxy, 2-Chlor-3-Nitro, 2-Chlor-3-Dimethylamino, 2-Chlor-3-Trifluormethoxy, 2-Chlor-3-Carbomethoxy, 2-Chlor-3-Trifluormethoxy, 2-Methyl-6-Fluor, 2-Methyl-6-Chlor, 2-Methyl-6-Ethyl, 2-Methyl-6-Methoxy, 2-Methyl-6-Dimethylamino, 2-Methyl-6-Trifluormethyl, 2-Methyl-6-Carbomethoxy, 2-Methyl-6-Nitro, 2-Methyl-5-Fluor, 2-Methyl-5-Chlor, 2-Methyl-5-Methoxy, 2-Methyl-5-Dimethylamino, 2-Methyl-5-Trifluormethyl, 2-Methyl-5-Carbomethoxy, 2-Methyl-6-Nitro, 2-Methyl-5-Trifluormethoxy, 2-Methyl-4-Fluor, 2-Methyl-4-Chlor, 2-Methyl-4-Methoxy, 2-Methyl-4-Dimethylamino, 2-Methyl-4-Trifluormethyl, 2-Methyl-4-Carbomethoxy, 2-Methyl-4-Nitro, 2-Methyl-4-Cyano, 2-Methyl-4-Sulfonylmethyl,2-Methyl-3-Fluor, 2-Methyl-3-Chlor, 2-Methyl-3-Methoxy, 2-Methyl-3-Dimethylamino, 2-Methyl-3-Trifluormethyl, 2-Methyl-3-Carbomethoxy, 2-Methyl-3-Nitro, 2-Methyl-3-Trifluormethoxy.

Verbindungen der Formel Ia mit $R^2$ = Trifluormethyl und Y bedeutet jeweils:
2-Methyl, 3-Methyl, 4-Methyl, 2-Ethyl, 3-Ethyl, 4-Ethyl, 2-Propyl, 3-Propyl, 4-Propyl, 2-i-Propyl, 3-i-Propyl, 4-i-Propyl, 2-n-Butyl, 3-n-Butyl, 4-n-Butyl, 2-s-Butyl, 3-s-Butyl, 4-s-Butyl, 2-t-Butyl, 3-t-Butyl, 4-t-Butyl, 2-Benzyl, 3-Benzyl, 4-Benzyl, 2-cyclo-Propyl, 3-cyclo-Propyl, 4-cyclo-Propyl, 2-Trifluormethyl, 3-Trifluormethyl, 4-Trifluormethyl, 2-Methoxy, 3-Methoxy, 4-Methoxy, 2-Ethoxy, 3-Ethoxy, 4-Ethoxy, 2-Trifluormethoxy, 3-Trifluormethoxy, 4-Trifluormethoxy, 2-Thiomethyl, 3-Thiomethyl, 4-Thiomethyl, 2-Fluor, 3-Fluor, 4-Fluor, 2-Chlor, 3-Chlor, 4-Chlor, 2-Brom, 3-Brom, 4-Brom, 2-Nitro, 3-Nitro, 4-Nitro, 2-Cyano, 3-Cyano, 4-Cyano, 2-Carbomethoxy, 3-Carbomethoxy, 4-Carbomethoxy, 2-Carboethoxy, 3-Carboethoxy, 4-Carboethoxy, 2-Carbamoyl, 3-Carbamoyl, 4-Carbamoyl, 2-N-Methylcarbamoyl, 3-N-Methylcarbamoyl, 4-N-Methylcarbamoyl, 2-N,N-Dimethylcarbamoyl, 3-N,N-Dimethylcarbamoyl, 4-N,N-Dimethylcarbamoyl, 2-N,N-Diethylcarbamoyl, 3-N,N-Diethylcarbamoyl, 4-N,N-Diethylcarbamoyl, 2-Amino, 3-Amino, 4-Amino, 2-N-Methylamino, 2-N-Methylamino, 4-N-Methylamino, 2-Dimethylamino, 3-Dimethylamino, 4-Dimethylamino, 2-N-Acetylamino, 3-N-Acetylamino, 4-N-Acetylamino, 2-Hydroxy, 3-Hydroxy, 4-Hydroxy,2-Phenyl, 3-Phenyl, 4-Phenyl, 2-Sulfonylmethyl, 3-Sulfonylmethyl, 4-Sulfonylmethyl, 3-t-Butoxy, 4-t-Butoxy, 3-O-$CH(CH_3)CO_2Me$, 4-O-$CH(CH_3)CO_2Me$, 3-N-Piperidyl, 4-N-Piperidyl, 4-N-Pyrrolidinyl, 3-Pyrrolidinyl, 3-N-Pyrrolyl, 4-N-Pyrrolyl, 2,3-Difluor, 2,4-Difluor, 2,5-Difluor, 2,6-Difluor, 2,3,4-Trifluor, 2,3,5-Trifluor, 2,3,6-Trifluor, 2,4,5-Trifluor, 2,4,6-Trifluor, 2,3-Dichlor, 2,4-Dichlor, 2,5-Dichlor, 2,6-Dichlor, 2,3,4-Trichlor, 2,3,5-Trichlor, 2,3,6-Trichlor, 2,4,5-Trichlor, 2,4,6-Trichlor, 2,3-Dimethyl, 2,4-Dimethyl, 2,5-Dimethyl, 2,6-Dimethyl, 2,3,4-Trimethyl, 2,3,5-Trimethyl, 2,3,6-Trimethyl, 2,4,5-Trimethyl , 2,4,6-Trimethyl, 2-Fluor-6-Methyl, 2-Fluor-6-Ethyl, 2-Fluor-6-Chlor, 2-Fluor-6-Methoxy, 2-Fluor-6-N,N-Dimethylamino, 2-Fluor-6-Trifluormethyl, 2-Fluor-6-Carbomethoxy, 2-Fluor-6-Nitro, 2-Fluor-5-Methyl, 2-Fluor-5-Chlor, 2-Fluor-5-Methoxy, 2-Fluor-5-N,N-Dimethylamino, 2-Fluor-5-Trifluormethyl, 2-Fluor-5-Carbomethoxy, 2-Fluor-5-Nitro, 2-Fluor-5-Trifluormethoxy,2-Fluor-4-Methyl, 2-Fluor-4-Chlor, 2-Fluor-4-Methoxy, 2-Fluor-4-N,N-Dimethylamino, 2-Fluor-4-Trifluormethyl, 2-Fluor-4-Carbomethoxy, 2-Fluor-4-Nitro, 2-Fluor-4-Cyano, 2-Fluor-4-Sulfonylmethyl,2-Fluor-3-Methyl, 2-Fluor-3-Chlor, 2-Fluor-3-Methoxy, 2-Fluor-3-N,N-Dimethylamino, 2-Fluor-3-Trifluormethyl, 2-Fluor-3-Carbomethoxy, 2-Fluor-3-Nitro, 2-Fluor-3-Trifluormethoxy, 2-Fluor-4-Chlor-5-Methoxy, 2-Chlor-6-Methyl, 2-Chlor-6-Ethyl, 2-Chlor-6-Fluor, 2-Chlor-6-Methoxy, 2-Chlor-6-Nitro, 2-Chlor-6-Dimethylamino, 2-Chlor-6-Trifluormethoxy, 2-Chlor-6-Carbomethoxy, 2-Chlor-5-Methyl, 2,4-Dichlor-5-Methyl, 2-Chlor-5-Fluor, 2,4-Dichlor-5-Fluor, 2-Chlor-5-Methoxy, 2-Chlor-5-Nitro, 2-Chlor-5-Dimethylamino, 2-Chlor-5-Trifluormethoxy, 2-Chlor-5-Carbomethoxy, 2-Chlor-5-trifluormethoxy, 2-Chlor-4-Methyl, 2-Chlor-4-Fluor, 2-Chlor-4,5-Difluor, 2-Chlor-4-Methoxy, 2-Chlor-4-Nitro, 2-Chlor-4-Dimethylamino, 2-Chlor-4-Trifluormethoxy, 2-Chlor-4-Carbomethoxy, 2-Chlor-4-Cyano, 2-Chlor-4-Sulfonylmethyl,2-Chlor-3-Methyl, 2-Chlor-3-Fluor, 2-Chlor-3-Methoxy, 2-Chlor-3-Nitro, 2-Chlor-3-Dimethylamino, 2-Chlor-3-Trifluormethoxy, 2-Chlor-3-Carbomethoxy, 2-Chlor-3-Trifluormethoxy, 2-Methyl-6-Fluor, 2-Methyl-6-Chlor, 2-Methyl-6-Ethyl, 2-Methyl-6-Methoxy, 2-Methyl-6-Dimethylamino, 2-Methyl-6-Trifluormethyl, 2-Methyl-6-Carbomethoxy, 2-Methyl-6-Nitro, 2-Methyl-5-Fluor, 2-Methyl-5-Chlor, 2-Methyl-5-Methoxy, 2-Methyl-5-Dimethylamino, 2-Methyl-5-Trifluormethyl, 2-Methyl-5-Carbomethoxy, 2-Methyl-6-Nitro, 2-Methyl-5-Trifluormethoxy, 2-Methyl-4-Fluor, 2-Methyl-4-Chlor, 2-Methyl-4-Methoxy, 2-Methyl-4-Dimethylamino, 2-Methyl-4-Trifluormethyl, 2-Methyl-4-Carbomethoxy, 2-Methyl-4-Nitro, 2-Methyl-4-Cyano, 2-Methyl-4-Sulfonylmethyl,2-Methyl-3-Fluor, 2-Methyl-3-Chlor, 2-Methyl-3-Methoxy, 2-Methyl-3-Dimethylamino, 2-Methyl-3-Trifluormethyl, 2-Methyl-3-Carbomethoxy, 2-Methyl-3-Nitro, 2-Methyl-3-Trifluormethoxy.

Bevorzugt sind weiterhin Verbindungen der Formel Ib,

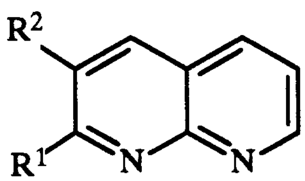

Ib

in der die Substituenten die folgende Bedeutung haben:

| | |
|---|---|
| $R^2$ = n-Propyl und $R^1$ bedeutet | Phenyl, 2-Chlorphenyl, 2-Fluorphenyl, 2,6-Difluorphenyl, 2-Methylphenyl, 2-Ethylphenyl und 2-Thienyl; |
| $R^2$ = n-Butyl und $R^1$ bedeutet | Phenyl, 2-Chlorphenyl, 2-Fluorphenyl, 2,6-Difluorphenyl, 2-Methylphenyl, 2-Ethylphenyl und 2-Thienyl; |
| $R^2$ = t-Butyl und $R^1$ bedeutet | Phenyl, 2-Chlorphenyl, 2-Fluorphenyl, 2,6-Difluorphenyl, 2-Methylphenyl, 2-Ethylphenyl und 2-Thienyl; |
| $R^2$ = s-Butyl und $R^1$ bedeutet | Phenyl, 2-Chlorphenyl, 2-Fluorphenyl, 2,6-Difluorphenyl, 2-Me- |

thylphenyl, 2-Ethylphenyl und 2-Thienyl;

| | |
|---|---|
| $R^2$ = Benzyl und $R^1$ bedeutet | Phenyl, 2-Chlorphenyl, 2-Fluorphenyl, 2,6-Difluorphenyl, 2-Methylphenyl, 2-Ethylphenyl und 2-Thienyl; |
| $R^2$ = Cyclopentyl und $R^1$ bedeutet | Phenyl, 2-Chlorphenyl, 2-Fluorphenyl, 2,6-Difluorphenyl, 2-Methylphenyl, 2-Ethylphenyl und 2-Thienyl; |
| $R^2$ = Cyclohexyl und $R^1$ bedeutet | Phenyl, 2-Chlorphenyl, 2-Fluorphenyl, 2,6-Difluorphenyl, 2-Methylphenyl, 2-Ethylphenyl und 2-Thienyl; |
| $R^2$ = Phenyl und $R^1$ bedeutet | Phenyl, 2-Chlorphenyl, 2-Fluorphenyl, 2,6-Difluorphenyl, 2-Methylphenyl, 2-Ethylphenyl und 2-Thienyl; |
| $R^2$ = Carboethoxy und $R^1$ bedeutet | Phenyl, 2-Chlorphenyl, 2-Fluorphenyl, 2,6-Difluorphenyl, 2-Methylphenyl, 2-Ethylphenyl und 2-Thienyl; |
| $R^2$ = Carbomethoxy und $R^1$ bedeutet | Phenyl, 2-Chlorphenyl, 2-Fluorphenyl, 2,6-Difluorphenyl, 2-Methylphenyl, 2-Ethylphenyl und 2-Thienyl; |
| $R^2$ = Cyano und $R^1$ bedeutet | Phenyl, 2-Chlorphenyl, 2-Fluorphenyl, 2,6-Difluorphenyl, 2-Methylphenyl, 2-Ethylphenyl und 2-Thienyl; |
| $R^2$ = Sulfonylmethyl und $R^1$ bedeutet | Phenyl, 2-Chlorphenyl, 2-Fluorphenyl, 2,6-Difluorphenyl, 2-Methylphenyl, 2-Ethylphenyl und 2-Thienyl; |
| $R^2$ = Sulfonylphenyl und $R^1$ bedeutet | Phenyl, 2-Chlorphenyl, 2-Fluorphenyl, 2,6-Difluorphenyl, 2-Methylphenyl, 2-Ethylphenyl und 2-Thienyl. |

Die Verbindungen I bzw. die sie enthaltenden herbiziden Mittel sowie deren umweltverträgliche Salze z.B. von Alkalimetallen und Erdalkalimetallen können als Herbizide in Kulturen wie Weizen, Reis, Mais, Soja und Baumwolle Unkräuter und Schadgräser sehr gut bekämpfen, ohne die Kulturpflanzen zu schädigen, ein Effekt, der vor allem auch bei niedrigen Aufwandmengen auftritt.

Die Verbindungen I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline oder deren Derivate, alkylierte Benzole oder deren Derivate, Methanol, Ethanol, Propanol, Butanol, Cyclohexanol, Cyclohexanon oder stark polare Lösungsmittel, wie N-Methylpyrrolidon oder Wasser in Betracht.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie

Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Formulierungen enthalten im allgemeinen zwischen 0,01 bis 95 Gew.-%, vorzugsweise zwischen 0,5 bis 90 Gew.-%, Wirkstoff. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen I können weiterhin beispielsweise wie folgt formuliert werden:

I. 20 Gew.-Teile der Verbindung Nr. 1.11 werden in einer Mischung gelöst, die aus 80 Gew.-Teilen alkyliertem Benzol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes von 40 Mol Ethylenoxid an 1 Mol Ricinusöl besteht. Durch Ausgießen und feines Verteilen der Lösung in 100 000 Gew.-Teilen Wasser erhält man eine wäßrige Dispersion, die 0,02 Gew.-% des Wirkstoffs enthält.

II. 20 Gew.-Teile der Verbindung Nr. 1.12 werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenyl und 10 Gew.-Teilen des Anlagerungsproduktes 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.-% des Wirkstoffs enthält.

III. 20 Gew.-Teile des Wirkstoffs Nr. 1.16 werden in einer Mischung gelöst, die aus 25 Gew.-Teilen Cyclohexanon, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinus besteht. Durch Eingießen und feines Verteilen der Lösung in 100 000 Gew.-Teilen Wasser erhält man eine wäßrige Dispersion, die 0,02 Gew.-% des Wirkstoffs enthält.

IV. 20 Gew.-Teile des Wirkstoffs Nr. 1.11 werden mit 3 Gew.-Teilen des Natriumsalzes der Diisobutylnaphthalin-α-sulfonsäure, 17 Gew.-Teile des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser enthält man eine Spritzbrühe, die 0,1 Gew.-% des Wirkstoffs enthält.

V. 3 Gew.-Teile des Wirkstoffs Nr. 1.12 werden mit 97 Gew.-Teilen feinteiligem Kaolin vermischt. Man erhält auf diese Weise ein Stäubemittel, das 3 Gew.-% des Wirkstoffs enthält.

VI. 20 Gew.-Teile des Wirkstoffs Nr. 1.16 werden mit 2 Gew.-Teilen Calciumsalz der Dodecylbenzolsulfonsäure, 8 Gew.-Teilen Fettalkohol-polyglykolether, 2 Gew.-Teilen Natriumsalz eines Phenol-Harnstoff-Formaldehyd-Kondensates und 68 Gew.-Teilen eines paraffinischen Mineralöls innig vermischt. Man erhält eine stabile ölige Dispersion.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 3,0, vorzugsweise 0,01 bis 1,0 kg/ha aktive Substanz (a.S.).

In Anbetracht der Vielseitigkeit der Applikationsmethoden können die erfindungsgemäßen Verbindungen I bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spp. altissima, Beta vulgaris spp. rapa, Brassica napus var. napus, Brassica napus var. napobrassica, Brassica rapa var. silvestris, Camellia sinensis, Carthamus tinctorius , Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spp., Manihot esculenta, Medicago sativa, Musa spp., Nicotiana tabacum (N. rustica), Olea europaea, Oryza sativa, Phaseolus lunatus, Phaseolus vulgaris, Picea abies, Pinus spp., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera, Zea mays.

Zur Verbreiterung des Wirkungsspektrums und zur Erzielung synergistischer Effekte können die Naphthyridine I mit zahlreichen Vertretern anderer herbizider oder wachstumsregulierender Wirkstoffgruppen gemischt und gemeinsam ausgebracht werden. Beispielsweise kommen als herbizide Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, die in 2-Stellung z.B. eine Carboxy- oder Carbimino-Gruppe tragen, Chinolincarbonsäurederivate, Imidazoline, Sulfonamide, Sulfonylharnstoffe, Aryloxy-, Heteroaryloxyphenoxypropionsäuren sowie deren Salze, Ester und Amide und andere in Betracht.

Außerdem kann es von Nutzen sein, die Verbindungen I allein oder in Kombination mit anderen Herbiziden oder Wachstumsregulatoren auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die Naphthyridine I eignen sich darüber hinaus als Fungizide.

Die Verbindungen I zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Speziell eignen sie sich zur Bekämpfung folgender Pflanzenkrankheiten: Erysiphe graminis (echter Mehltau) in Getreide, Erysiphe cichoracearum und Sphaerotheca fuliginea an Kürbisgewächsen, Podosphaera leucotricha an Äpfeln, Uncinula necator an Reben, Puccinia-Arten an Getreide, Rhizoctonia-Arten an Baumwolle und Rasen, Ustilago-Arten an Getreide und Zuckerrohr, Venturia inaequalis (Schorf) an Äpfeln, Helminthosporium-Arten an Getreide, Septoria nodorum an Weizen, Botrytis cinerea (Grauschimmel) an Erdbeeren, Reben, Cercospora arachidicola an Erdnüssen, Pseudocercosporella herpotrichoides an Weizen, Gerste, Pyricularia oryzae an Reis, Phytophthora infestans an Kartoffeln und Tomaten, Fusa7rium- und Verticillium-Arten an verschiedenen Pflanzen, Plasmopara viticola an Reben, Alternaria-Arten an Gemüse und Obst.

Die Verbindungen I werden angewendet, indem man die Pilze oder die vor Pilzbefall zu schützenden Pflanzen, Saatguter, Materialien oder den Erdboden mit einer fungizid wirksamen Menge der Wirkstoffe behandelt. Die Anwendung erfolgt vor oder nach der Infektion der Materialien, Pflanzen oder Samen durch die Pilze.

Sie können in die üblichen Formulierungen überführt werden, wie Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung des ortho-substituierten Benzylesters einer Cyclopropancarbonsäure gewährleisten. Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Beispiele

Beispiel 1: 2-Phenyl-3-isopropyl-1,8-naphthyridin

2-Amino-nikotinaldehyd (1,5 g, 12,3 mmol) und Isobutylphenylketon (2,09 g, 12,9 mmol) werden in 12 ml Methanol gelöst und bei Raumtemperatur mit 1,5 ml 40 % KOH tropfenweise versetzt. Es wird für 5 h zum Rückfluß erhitzt. Nach wäßriger Aufarbeitung wird das Rohprodukt durch Säulenchromatographie über $SiO_2$ (Cyclohexan/Essigester 2:8) gereinigt. Man erhält 600 mg (20 % Ausbeute) der gewünschten Verbindung.
Schmp. 165-166 °C

Beispiel 2: 2-(2'-Fluorphenyl)-1,8-naphthyridin

2-Amino-nikotinaldehyd (1,0 g, 8,2 mmol) und 2-Fluoracetophenon (1,19 g, 8,6 mmol) werden in 8ml MeOH gelöst und bei Raumtemperatur tropfenweise mit 40 % wäßr. KOH versetzt. Es wird über Nacht gerührt, anschließend auf Eis gegossen und das Produkt wird abgesaugt. Man erhält 1,36 g (74 % Ausbeute) des gewünschten Produkts.
Schmp. 111-114 °C

Beispiel 3: 2-(2',6'-Difluorphenyl)-1,8-naphthyridin

2-Amino-nikotinaldehyd (2,44 g, 20 mmol) und 2,6-Difluoracetophenon (3,28 g, 21 mmol) werden in 20 ml MeOH gelöst. Es wird tropfenweise mit 3 ml 40 % KOH versetzt. Nach Abklingen der exothermen Reaktion wird auf Wasser gegossen und der ausgefallene Rückstand abgesaugt. Man erhält 3,8g (79 % Ausbeute) des gewünschten Produkts.
Schmp. 100-103 °C

Beispiel 4: 2-(2'-Trifluormethyl)-1,8-naphthyridin

2-Amino-nikotinaldehyd (2,44 g, 20 mmol) und 2-Trifluoracetophenon (3,95 g, 21 mmol) werden in 20 ml MeOH gelöst und bei Raumtemperatur mit 3 ml 40 % wäßr. KOH versetzt. Nach 1 h wird auf Wasser gegossen und der ausgefallene Feststoff abgesaugt. Nach Waschen mit Ether und Trocknen i. Vak. erhält man 3,56 g (61 % Ausbeute) 1-(2'-Trifluormethylphenyl)-3-(2-amino-3-pyridyl)-2-propen-1-on.
Schmp. 161-164 °C.

Die oben erhaltene Verbindung (2,7 g) wird in Eisessig (20 ml) gelöst, mit konz. $H_2SO_4$ (2 ml) versetzt und 8 h refluxiert. Nach Abkühlen wird mit NaOH neutralisiert, mit Ethylacetat extrahiert und das erhaltene Rohprodukt über $SiO_2$ (Cyclohexan/Ethylacetat 1:1) chromatographisch gereinigt. Man erhält 1,95 g (77 % Ausbeute) des Naphthyridins.
Schmp. 88-91 °C.

Beispiel 5: 2-(2'Chlorphenyl)-6-methyl-7-phenyl-1,8-Naphthyridin

2-Amino-5-Methyl-6-phenyl-nikotinaldehyd (1,5 g, 7,1 mmol) und 2-Chloracetophenon (1,2 g, 7,7 mmol) werden in 10 ml MeOH gelöst, bei Raumtemperatur mit 2 ml 40 % KOH und über Nacht bei dieser Temperatur gerührt. Der ausgefallene Niederschlag wird durch Chromatographie ($SiO_2$, EtOAc/MeOH 9:1) gereinigt. Man erhält 0,9 g (Ausbeute 38 %) der gewünschten Verbindung.
Schmp. 219-222 °C

Beispiel 6: 2-(2'-Chlorphenyl)-4,6-dimethyl-7-phenyl-1,8-naphthyridin

2-Acetyl-2-Amino-5-methyl-6-phenylpyridin (0,9 g, 3,9 mmol) und 2-Chloracetophenon (0,7 g, 4,6 mmol) werden in 20 ml MeOH gelöst und mit 2 ml 40 % KOH versetzt. Nach 3 tägigem Rühren bei Raumtemperatur wird der ausgefallene Niederschlag abgesaugt und chromatographisch gereinigt ($SiO_2$, Cyclohexan/EtOAc 9:1). Man erhält 0,4 g (Ausbeute 30 %) der gewünschten Verbindung.
Schmp. 151-153 °C

Beispiel 7: 2-(2-Thiazolyl)-1,8-Naphthyridin

2-Amino-nikotinaldehyd (1,5 g, 12,3 mmol) und 2-Acetylthiazol (1,64 g, 12,9 mmol) werden in 12ml MeOH gelöst und mit 1,5 ml 40 % KOH versetzt. Nach 3 h ist die Reaktion beendet und es wird auf Wasser gegossen. Der ausgefallene Feststoff wird abgesaugt, gewaschen und getrocknet. Man erhält 1,22 g (Ausbeute 46 %) des gewünschten Produkts.
Schmp. 160-165 °C
Weitere Verbindungen, die in analoger Weise erhalten wurden, sind den nachfolgenden Tabellen zu entnehmen.

Tabelle 1

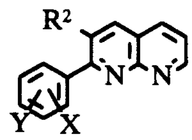

| Nr. | X, Y | $R^2$ | Schmp. |
|---|---|---|---|
| 1.1 | H, H | H | 113-115[a)] |
| 1.2 | 4-$CH_3$, H | H | 146-150[b)] |
| 1.3 | 4-Cl, H | H | 197-201[b)] |
| 1.4 | 4-$OCH_3$, H | H | 145-150[b)] |
| 1.5 | 3-Cl, H | H | 100-102 |
| 1.6 | 3-$CH_3$, H | H | 101-104 |
| 1.7 | 3-$CF_3$, H | H | 142-147[e)] |
| 1.8 | 3-$OCH_3$, H | H | 136-139 |
| 1.9 | 3,4-$Cl_2$, H | H | 199-202 |
| 1.10 | H, H | $CH_3$ | 107-111[c)] |
| 1.11 | 2-$CH_3$, H | H | 128-130 |
| 1.12 | 2-Cl, H | H | 137-140 |
| 1.13 | H, H | i-$C_3H_7$ | 165-167 |
| 1.14 | 2-$OCH_3$, H | H | 137-141[d)] |
| 1.15 | 2-F, H | H | 111-114 |
| 1.16 | H, H | Et | 123-125[c)] |
| 1.17 | H, H | 2-Ethylhexyl | 75-78 |
| 1.18 | H, H | n-$C_3H_7$ | 59-64 |
| 1.19 | 4-F, H | H | 208-209 |
| 1.20 | 4-$CF_3$, H | H | 179 |
| 1.21 | 4-CN, H | H | 224-226 |
| 1.22 | 2-$NO_2$, H | H | 188-190 |
| 1.23 | 2-Br, H | H | 131-136 |
| 1.24 | 2-OH, H | H | 188-191[b)] |
| 1.25 | 2-OH, H | $CH_3$ | 117-119 |
| 1.26 | 2-$CH_3$, 5-t-$C_4H_9$ | $CH_3$ | 184-186 |
| 1.27 | 3-$CF_3$, H | H | 88-91 |
| 1.28 | 2,6-$Cl_2$ | H | 150-156 |
| 1.29 | 2-F, H | $CH_3$ | 65-70 |
| 1.30 | 2,6-$F_2$ | H | 100-103 |
| 1.31 | 2-$CF_3$, H | $CH_3$ | 214-219 |
| 1.32 | 2-F, 6-$CF_3$ | H | 118 |

[a)-e),i)] nicht erfindungsgemäße Verbindungen, siehe Literaturangaben am Ende der Tabellen

| Nr. | X, Y | $R^2$ | Schmp. |
|---|---|---|---|
| 1.33 | 2,6-$F_2$ | $CH_3$ | 74-77 |
| 1.34 | 3-$OCF_3$, H | H | 105-109 |
| 1.35 | 2-F, 4-$OCH_3$ | H | 126-129 |
| 1.36 | 2,5-$F_2$ | H | 119-125 |
| 1.37 | 2,3-$F_2$ | $CH_3$ | 140-141 |
| 1.38 | 2,6-$F_2$ | $C_2H_5$ | 119-120 |
| 1.39 | 2,4-$F_2$ | H | 151 |
| 1.40 | 2-F, 3-$CF_3$ | $CH_3$ | 189-190 |
| 1.41 | 2-F, 3-$CF_3$ | H | 145-146 |
| 1.42 | 2,3-$F_2$ | H | 131 |
| 1.43 | 2,5-$F_2$ | $CH_3$ | 130 |
| 1.44 | 2,4-$F_2$ | $CH_3$ | 157-159 |
| 1.45 | 4-$NO_2$, H | H | > 250 |
| 1.46 | 2-$CH_3$, H | $CH_3$ | 124 |
| 1.47 | 2-F, H | $C_2H_5$ | 91 |
| 1.48 | 2-$CH_3$, H | $C_2H_5$ | 128 |
| 1.49 | 2,3-$Cl_2$ | H | 190-192 |
| 1.50 | 2,4-$Cl_2$ | H | 169-174 |
| 1.51 | 2,5-$Cl_2$ | H | 139 |
| 1.52 | 2,3,4-$Cl_3$ | H | 178-181 |
| 1.53 | 2-$CH_3$, 4-$CH_3$ | H | 100-104 |
| 1.54 | 2-$NH_2$ | H | 200-206 |
| 1.55 | H,H | 3-Pyridyl | 150 |
| 1.56 | H,H | $SO_2$-Phenyl | 183-184 |
| 1.57 | H,H | C(O)-Phenyl | 182-183[1)] |
| 1.58 | H,H | $CO_2C_2H_5$ | 106-112 |
| 1.59 | 2-Cl | $CH_3$ | 140-142 |
| 1.60 | 2-$OCH_3$ | $CH_3$ | 146-149 |
| 1.61 | 2-,3-,6-$F_3$ | H | 133-136 |
| 1.62 | 2-Cl | $C_2H_5$ | 135-136 |
| 1.63 | NHAcetyl | H | 169-170 |
| 1.64 | 2-F | i-$C_3H_7$ | 141 |
| 1.65 | 2-$CH_3$ | i-$C_3H_7$ | 131 |
| 1.66 | 2-Cl, 5-$NO_2$ | H | 175-179 |
| 1.67 | 2-OC(O)$C_6H_5$ | H | 127 |
| 1.68 | 2-$CH_3$, 4-$CH_3$, 5-$CH_3$ | H | 98-106 |
| 1.69 | 2-OC(O)$CH_3$ | H | 120 |
| 1.70 | 2-$CH_3$, 4-$CH_3$, 6-$CH_3$ | H | 130-132 |

| Nr. | X, Y | $R^2$ | Schmp. |
|---|---|---|---|
| 1.71 | 2-Cl | i-$C_3H_7$ | 132-133 |
| 1.72 | 2-F, 4-Cl, 5-F | H | 174-177 |
| 1.73 | 2-$CH_3$, 3-$CH_3$, 6-$CH_3$ | H | 133-134 |
| 1.74 | H, H | CN | 225-226 |
| 1.75 | H, H | $CH_2OCH_3$ | 94-97 |
| 1.76 | 2-$C_2H_5$ | $CH_3$ | 145-148 |

i) nicht erfindungsgemäße Verbindungen, siehe Literaturangaben am Ende der Tabellen

Tabelle 2

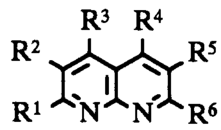

| Nr. | $R^1$ | $R^2$ | $R^3$ | $R^4$ | $R^5$ | $R^6$ | Schmp. |
|---|---|---|---|---|---|---|---|
| 2.1 | Phenyl | H | H | H | H | i-$C_3H_7$ | - |
| 2.2 | Phenyl | $CH_3$ | H | H | H | i-$C_3H_7$ | - |
| 2.3 | 2-Thienyl | H | H | H | H | i-$C_3H_7$ | 110-112 |
| 2.4 | 3-Thienyl | H | H | H | H | i-$C_3H_7$ | 102-103 |
| 2.5 | 2-Cl-Phenyl | H | H | H | H | i-$C_3H_7$ | 110-112 |
| 2.6 | Phenyl | H | H | $CH_3$ | H | $CH_3$ | 152-154 |
| 2.7 | Phenyl | $CH_3$ | H | $CH_3$ | H | $CH_3$ | 127-130 |
| 2.8 | 2-Thienyl | H | H | $CH_3$ | H | $CH_3$ | 135-139 |
| 2.9 | 3-Thienyl | H | H | $CH_3$ | H | $CH_3$ | 119-122 |
| 2.10 | 2-Cl-Phenyl | H | H | $CH_3$ | H | $CH_3$ | 140-141 |
| 2.11 | Phenyl | $CH_3$ | H | H | H | Phenyl | 165-169 |
| 2.12 | Phenyl | H | H | H | H | Phenyl | 204-207[f)] |
| 2.13 | Phenyl | $CH_3$ | H | $CH_3$ | H | Phenyl | 193-195 |
| 2.14 | Phenyl | $CH_3$ | H | H | $CH_3$ | Phenyl | 240-248 |
| 2.15 | 2-Thienyl | H | H | H | H | Phenyl | 201-203 |
| 2.16 | 2-Thienyl | H | H | H | $CH_3$ | Phenyl | 257-258 |
| 2.17 | 2-Thienyl | $CH_3$ | H | H | H | Phenyl | 251-253 |
| 2.18 | 2-Cl-Phenyl | H | H | H | $CH_3$ | Phenyl | 219-222 |
| 2.19 | 2-Thienyl | H | H | H | H | 2-Thienyl | 224-225 |
| 2.20 | 2-Cl-Phenyl | H | H | H | H | 2-Thienyl | 204-205 |
| 2.21 | 2-Thienyl | H | $CH_3$ | H | $CH_3$ | Phenyl | 243-244 |
| 2.22 | 2-Cl-Phenyl | H | $CH_3$ | H | $CH_3$ | Phenyl | 151-153 |
| 2.23 | 2-F-Phenyl | $CH_3$ | H | $CH_3$ | H | $CH_3$ | 131-132 |
| 2.24 | 2-F-Phenyl | $CH_3$ | H | H | H | i-$C_3H_7$ | 102-103 |
| 2.25 | Phenyl | $C_2H_5$ | H | $CH_3$ | H | $CH_3$ | 86- 88 |
| 2.26 | Phenyl | $C_2H_5$ | H | H | H | i-$C_3H_7$ | 124-125 |
| 2.27 | Phenyl | H | H | H | $CH_3$ | H | 129-131 |
| 2.28 | Phenyl | $CH_3$ | H | H | $CH_3$ | H | 175-177 |
| 2.29 | Phenyl | $C_2H_5$ | H | H | $CH_3$ | H | 81-82 |
| 2.30 | 2-F-Phenyl | $CH_3$ | H | H | $CH_3$ | H | 99-102 |
| 2.31 | Phenyl | H | H | $CH_3$ | H | H | 110-111 |
| 2.32 | Phenyl | $CH_3$ | H | $CH_3$ | H | H | 150-152 |

f) nicht erfindungsgemäße Verbindungen, siehe Literaturangaben am Ende der Tabellen

| Nr. | $R^1$ | $R^2$ | $R^3$ | $R^4$ | $R^5$ | $R^6$ | Schmp. |
|---|---|---|---|---|---|---|---|
| 2.33 | Phenyl | $C_2H_5$ | H | $CH_3$ | H | H | 98-100 |
| 2.34 | 2-F-Phenyl | $CH_3$ | H | $CH_3$ | H | H | 148-149 |
| 2.35 | 2-,6-$F_2$-Phenyl | $CH_3$ | H | $CH_3$ | H | H | 179-180 |
| 2.36 | 2-,6-$F_2$-Phenyl | $CH_3$ | H | H | $CH_3$ | H | 152-153 |
| 2.37 | 2-F-Phenyl | $CH_3$ | H | H | H | $CH_3$ | 137 |
| 2.38 | Phenyl | $CH_3$ | H | H | H | $CH_3$ | 132 |
| 2.39 | Phenyl | $C_2H_5$ | H | H | H | $CH_3$ | 119-122 |
| 2.40 | 2-,6-$F_2$-Phenyl | $CH_3$ | H | H | H | $CH_3$ | 170 |
| 2.41 | Phenyl | H | $CH_3$ | H | H | H | 117-119 |
| 2.42 | Phenyl | H | H | $CF_3$ | H | $CH_3$ | 99-100 |
| 2.43 | 2-F-Phenyl | $CH_3$ | $CH_3$ | H | H | H | 139-141 |
| 2.44 | 2-F-Phenyl | $CH_3$ | H | $CF_3$ | H | $CH_3$ | 150-151 |
| 2.45 | 2-,6-$F_2$-Phenyl | $CH_3$ | H | $CF_3$ | H | $CH_3$ | 120-122 |
| 2.46 | 3-Pyridyl | Phenyl | H | H | H | H | 150 |
| 2.47 | (2-Amino)-3-Pyridyl | H | $CH_3$ | H | H | H | 224-227 |

Tabelle 3

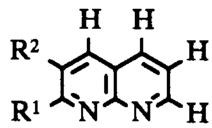

| Nr. | $R^1$ | $R^2$ | Schmp. |
|---|---|---|---|
| 3.1 | 2-Thienyl | H | 114-119[b] |
| 3.2 | 2-Pyridyl | H | 147 |
| 3.3 | 3-Thienyl | H | 101-108 |
| 3.4 | 2-Thienyl | $CH_3$ | 101-103 |
| 3.5 | 2-(5-Chlorthienyl) | H | 212-214 |
| 3.6 | 2-Furanyl | H | 137-140[b] |
| 3.7 | 4-Pyridyl | H | 169-171[b] |
| 3.8 | 3-Pyridyl | H | 144 |
| 3.9 | N-Methyl-2-pyrrolyl | H | 115-119 |
| 3.10 | 2-(3-Methylthienyl) | H | 97-103 |
| 3.11 | 2-Thiazolyl | H | 160-165 |
| 3.12 | 5-(2,4-Dimethylthiazolyl) | H | 130-132 |
| 3.13 | 3-(2,5-Dimethyl)thienyl | H | 80-83 |
| 3.14 | 2-Naphthyl | H | 133-136[b] |

Tabelle 4

| Nr. | | Schmp. |
|---|---|---|
| 4.1 | 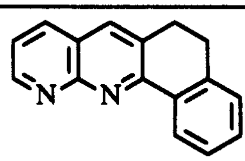 | 76-88[g] |
| 4.2 | 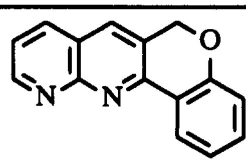 | 201-202[h] |
| 4.3 | 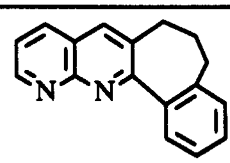 | 134-137[g] |
| 4.4 | 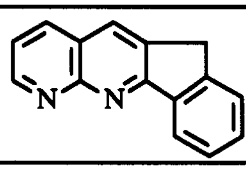 | 201-203[g] |

[b], [g], [h] nicht erfindungsgemäße Verbindungen, siehe Literaturangaben am Ende der Tabellen

Literaturangaben zu Tabellen 1 - 4

[a] E.M. Hawes, D.G. Wibberley, J. Chem. Soc.(C) 1967, 1564
[b] K.V. Reddy et al., J. Ind. Chem. Soc. 1986, 443

[c] T. Higashino et al., Chem. Pharm. Bull 1978, 26, 3242
[d] Yakugaku Zasshi 1979, 99, 451-7 (CA 92(19): 163866a)
[e] D.K. Garecki, E.M. Hawes, J. Med. Chem. 1977, 20(1), 124
[f] J.P. Sauvage et al., Tetrahedron Lett. 1982, 23, 5291
[g] R.P. Thummel et al., J. Heterocycl. Chem. 1986, 23, 689
[h] E.J.S. Reddy et al., Natl. Acad. Sci. Lett. (India) 1985, 8, 19 (CA 103: 51079k)
[i] G. Rama Rao et al., Ind. J. Chem. 1988, 27B, 200.

Anwendungsbeispiele

Die herbizide Wirkung der Naphthyridine der Formel I ließ sich durch Gewächshausversuche zeigen:
Als Kulturgefäße dienten Plastiktöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde. Die Aufwandmenge für die Vorauflaufanwendung betrug 2,0 kg/ha a.S.

Zum Zweck der Nachauflaufbehandlung wurden die Testpflanzen je nach Wuchsform erst bis zu einer Wuchshöhe 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachlaufanwendung betrug 2,0 bzw. 1,0 kg/ha a.S.

Die Pflanzen wurden artenspezifisch bei Temperaturen von 10 - 25 °C bzw. 20 - 35 °C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Die herbizide Wirkung wurde nach einer Skala von 0 bis 100 bewertet. Dabei bedeutet 100 kein Aufgang der Pflanzen bzw. völlige Zerstörung zumindest der oberirdischen Teile und 0 keine Schädigung oder normaler Wachstumsverlauf.

Die wachstumsregulierende Wirkung wurde durch Längenmessung bestimmt. Bei Versuchsende wurden die Wuchshöhen der behandelten Pflanzen gemessen und zur Wuchshöhe von nicht behandelten Pflanzen in Beziehung gesetzt.

Die in den Gewächshausversuchen verwendeten Pflanzen setzten sich aus folgenden Arten zusammen:

| Lateinischer Name | Deutscher Name | Englischer Name |
|---|---|---|
| Alopecurus myosuroides | Ackerfuchsschwanz | blackgrass |
| Amaranthus retroflexus | Zurückgekrümmter Fuchsschwanz | redroot pigweed |
| Avena fatua | Flughafer | wild oat |
| Chenopodium album | Weißer Gänsefuß | lambsquarters (goosefoot) |
| Echinochloa crus-galli | Hühnerhirse | barnyardgrass |
| Ipomoea spp. | Prunkwindearten | morningglory |
| Setaria faberii | Borstenhirse | giant foxtail |
| Setaria viridis | Grüne Borstenhirse | green foxtail |

Die Ergebnisse zeigten eine sehr gute herbizide Wirkung der erfindungsgemäßen Verbindungen Nr. 1.11, 1.12, 1.29 und 1.16.

**Patentansprüche**

1. Substituierte Naphthyridine der allgemeinen Formel I,

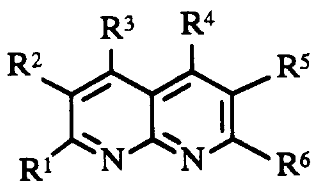

I

in der die Substituenten folgende Bedeutung haben:

$R^1$

(a) ein ggf. substituierter fünfgliedriger Heteroaromat mit ein bis zwei Heteroatomen, ausgewählt aus Stickstoff, Sauerstoff oder Schwefel, wobei der Heteroaromat benzokondensiert sein kann;

(b) ein ggf. substituierter sechsgliedriger Heteroaromat enthaltend ein bis zwei Stickstoffatome;

(c) Phenyl, substituiert mit ein bis vier Substituenten, ausgewählt aus der Gruppe $C_1$-$C_8$-Alkyl, $C_2$-$C_8$-Alkenyl, $C_2$-$C_8$-Alkinyl,$C_3$-$C_8$-Cycloalkyl, Phenyl, $C_1$-$C_5$-Alkoxy, $C_1$-$C_5$-Thioalkyl, ein fünf -oder sechsgliedriger gesättigter Heterocyclus, ein fünf -oder sechsgliedriger aromatischer Heterocyclus, wobei alle voranstehend genannten Substituenten jeweils noch selber ein- bis dreimal substituiert sein können, Hydroxy, eine Gruppe $OC(O)R^{14}$, Amino, eine Gruppe $NR^{15}R^{16}$, Halogen, Cyano, Nitro, Carboxyl, eine Gruppe $R^7SO_2$-, -$C(O)R^8$, -$C(Y)R^9$, wobei Y für Sauerstoff oder eine Gruppe $NR^{13}$ steht, -$C(VR^{10}WR^{11})R^{12}$, wobei V und W unabhängig voneinander für O oder S stehen;

| | |
|---|---|
| $R^7$ | $C_1$-$C_8$-Alkyl, $C_3$-$C_8$-Cycloalkyl, $C_1$-$C_8$-Halogenalkyl, $C_1$-$C_5$-Alkoxy, Phenyl, Naphthyl, Aryl($C_1$-$C_6$-)alkyl, Heteroaryl, Hydroxy, Amino, $C_1$-$C_8$-Monoalkyl- oder $C_1$-$C_8$-Dialkylamino, $C_5$-$C_8$-Cycloalkylamino; |
| $R^8$ | Hydroxy, Amino, $C_1$-$C_8$-Monoalkyl- oder $C_1$-$C_8$-Dialkylamino, $C_5$-$C_8$-Cycloalkylamino, $C_1$-$C_6$-Alkylthio, $C_1$-$C_8$-Alkoxy; |
| $R^9$ | Wasserstoff, $C_1$-$C_8$-Alkyl, $C_3$-$C_8$-Cycloalkyl, $C_1$-$C_8$-Halogenalkyl, $C_1$-$C_4$-Alkoxy($C_1$-$C_6$-)alkyl, $C_1$-$C_8$-Aminoalkyl, Phenyl, Aryl($C_1$-$C_6$-)alkyl, Heteroaryl; |
| $R^{10}$, $R^{11}$ | $C_1$-$C_8$-Alkyl, $C_1$-$C_6$-Halogenalkyl, $C_1$-$C_4$-Alkoxy($C_1$-$C_6$-)alkyl oder $R^{10}$ und $R^{11}$ bilden zusammen eine Di-, Tri-, oder Tetramethylenkette die ggf. durch ein bis zwei $C_1$-$C_8$-Alkyl-, $C_1$-$C_6$-Halogenalkyl-, $C_1$-$C_4$-Alkoxy($C_1$-$C_6$-)alkylgruppen oder durch eine Oxogruppe substituiert ist; |
| $R^{12}$ | Wasserstoff, $C_1$-$C_8$-Alkyl, $C_3$-$C_8$-Cycloalkyl, $C_1$-$C_8$-Halogenalkyl, $C_1$-$C_4$-Alkoxy($C_1$-$C_6$-)alkyl, $C_1$-$C_8$-Aminoalkyl, Phenyl, Aryl($C_1$-$C_6$-)alkyl, Heteroaryl; |
| $R^{13}$ | $C_1$-$C_8$-Alkyl, $C_2$-$C_8$-Alkenyl, $C_3$-$C_8$-Cycloalkyl, $C_1$-$C_8$-Halogenalkyl, $C_1$-$C_4$-Alkoxy($C_1$-$C_6$-)alkyl, $C_1$-$C_8$-Aminoalkyl, Phenyl, Aryl($C_1$-$C_6$-)alkyl, Heteroaryl, Hydroxy, ggf. substituiertes $C_6$-$C_{10}$-Aryloxy, ggf. substituiertes $C_1$-$C_8$-Alkoxy, Amino, $C_1$-$C_8$-Monoalkyl- oder $C_1$-$C_8$-Dialkylamino, $C_5$-$C_8$-Cycloalkylamino, ggf. substituiertes $C_6$-$C_{10}$-Arylamino; |
| $R^{14}$ | $C_1$-$C_5$-Alkyl, ggf. substituiertes Phenyl, $C_1$-$C_5$-Alkoxy, $C_1$-$C_5$-Halogenalkoxy, Benzyloxy, Amino, Alkylamino, Dialkylamino, ein gesättigter über N gebundener fünf- oder sechsgliedriger Heterocyclus, der noch ein weiteres Heteroatom N oder O enthalten kann; |
| $R^{15}$ | Wasserstoff, $C_1$-$C_5$-Alkyl, ggf. substituiertes Phenyl; |
| $R^{16}$ | Wasserstoff, $C_1$-$C_5$-Alkyl, ggf. substituiertes Phenyl, eine Gruppe -$C(O)R^{14}$ oder $R^{16}$ bildet gemeinsam mit $R^{15}$ eine vier- oder fünfgliedrige Methylenkette, die ihrerseits mit ein oder zwei Methylgruppen substituiert sein kann und in der eine Methylengruppe durch Sauerstoff ersetzt sein kann; |

(d) Phenyl, in dem zwei direkt benachbarte Positionen über eine Tri- oder Tetramethylengruppe, welche ihrerseits ein bis drei $C_1$-$C_3$-Alkyl-, $C_1$-$C_2$-Alkoxy- und/oder Halogensubstituenten tragen kann, miteinander verbunden sind und die übrigen Positionen unsubstituiert oder durch einen der unter (c) aufgeführten Phenylsubstituenten substituiert sind;

(e) Phenyl, das über die ortho-Position mit $R^2$ verbunden ist zu einer Kette aus ein bis vier Methylengruppen oder ein bis drei Methylengruppen und einem Sauerstoffatom, wobei jede Methy-

lengruppen ein oder zwei $C_1$-$C_3$-Alkylreste tragen kann;

$R^2$-$R^6$

Wasserstoff, $C_1$-$C_8$-Alkyl, $C_2$-$C_8$-Alkenyl, $C_3$-$C_8$-Cycloalkyl, $C_1$-$C_8$-Halogenalkyl, $C_1$-$C_4$-Alkoxy($C_1$-$C_6$)-alkyl, $C_1$-$C_8$-Aminoalkyl, ggf. substituiertes Phenyl, Benzyl, ggf. substituiertes über einen Kohlenstoff verknüpftes fünf- oder sechsgliedriges Heteroaryl, Cyano, Nitro, Carboxyl, Sulfonylmethyl, $C_1$-$C_4$-Alkoxycarbonyl, Carbamoyl, $C_1$-$C_8$-Alkylcarbamoyl, Dialkylcarbamoyl, $C_1$-$C_4$-Alkanoyl, Benzoyl oder $R^4$ und $R^5$ oder $R^5$ und $R^6$ bilden gemeinsam eine Kette $CH_2$-$(CH_2)_n$-$CH_2$ mit n = 1 bis 3 oder einen unkondensierten aromatischen Ring;

mit der Maßgabe, daß Verbindungen mit folgenden Substituentenkombinationen ausgenommen sind:

i) wenn $R^1$ = Phenyl und $R^3$-$R^6$ = Wasserstoff, steht $R^2$ nicht für Wasserstoff, Methyl, Ethyl, n-Propyl, Phenyl oder Chlor;

ii) wenn $R^1$ = Phenyl und $R^2$-$R^5$ = Wasserstoff, steht $R^6$ nicht für Phenyl oder Methyl;

iii) wenn $R^1$ = Phenyl und $R^2$-$R^4$ sowie $R^6$ = Wasserstoff, steht $R^5$ nicht für Phenyl oder Benzoyl;

iv) wenn $R^1$ = Phenyl, $R^2$-$R^4$ = Wasserstoff und $R^6$ = Methyl, steht $R^5$ nicht für Acetyl oder Ethoxycarbonyl;

v) wenn $R^1$ = Phenyl, $R^3$ = Chlor, $R^2$; $R^4$ und $R^5$ = Wasserstoff, steht $R^6$ nicht für Methyl;

vi) wenn $R^1$ = Phenyl, $R^2$ = Cyano und $R^3 + R^5$ = Wasserstoff, stehen $R^4$ und $R^6$ nicht gleichzeitig für Methyl;

vii) wenn $R^2$-$R^6$ Wasserstoff,

steht $R^1$ nicht für 2-Pyridyl, 3-Pyridyl, 4-Pyridyl, 2-Thienyl, 2-Furanyl, 1-Naphthyl, 2-Naphthyl, Phenyl, 4-Chlorphenyl, 4-Bromphenyl, 4-Nitrophenyl, 4-Hydroxyphenyl, 4-Methoxyphenyl, 4-Methylphenyl, p-Biphenyl, 2-Hydroxy-4-chlorphenyl, 3-Nitrophenyl, 3-Trifluormethylphenyl, 2-Hydroxyphenyl, 2-Methoxyphenyl, 2-Hydroxy-5-nitrophenyl, 2-Hydroxy-5-methylphenyl;

viii) 6-H-Indeno-[1,2-b][1.8]naphthyridin, 5,6-Dihydronaphto[1.2-b][1.8]naphthyridin, 6H-[1]-benzopyrano[4.3-b][1.8]naphthyridin oder 6,7-Dihydro5H-benzo[6.7]cyclohepta[1,2-b][1.8]-naphthyridin.

**2.** Naphthyridine der Formel I gemäß Anspruch 1, in der die Substituenten $R^2$ bis $R^6$ der in Anspruch 1 genannte Bedeutung haben und $R^1$ wie folgt definiert ist:

$R^1$

Phenyl oder Phenyl, substituiert mit ein bis vier Substituenten ausgewählt aus:

$C_1$-$C_8$-Alkyl, ggf. substituiert mit Halogen, $C_1$-$C_4$-Alkoxy, Amino, $C_1$-$C_4$-Alkylamino, $C_1$-$C_4$-Dialkylamino, $C_1$-$C_4$-Thioalkyl, Phenyl, Phenoxy, Thiophenyl, Aminophenyl, $C_1$-$C_4$-Alkylaminophenyl, wobei die fünf letztgenannten Substituenten im Phenylring ein bis drei Substituenten, ausgewählt aus $C_1$-$C_4$-Alkoxy, Halogen, $C_1$-$C_4$-Alkyl oder Nitro tragen können;

$C_2$-$C_8$-Alkenyl oder $C_2$-$C_8$-Alkinyl, jeweils ggf. substituiert mit Halogen, $C_1$-$C_4$-Alkoxy, $C_1$-$C_4$-Thioalkyl, Phenyl, Phenoxy, Thiophenyl, wobei die fünf letztgenannten Substituenten im Phenylring ein bis drei Substituenten, ausgewählt aus $C_1$-$C_4$-Alkoxy, Halogen, $C_1$-$C_4$-Alkyl oder Nitro tragen können;

$C_3$-$C_8$-Cycloalkyl, ggf. substituiert mit Halogen, $C_1$-$C_4$-Alkoxy, Amino, $C_1$-$C_4$-Alkylamino, $C_1$-$C_4$-Dialkylamino, $C_1$-$C_4$-Thioalkyl, Phenyl, Phenoxy, Thiophenyl, Aminophenyl, $C_1$-$C_4$-Alkylaminophenyl, wobei die fünf letztgenannten Substituenten im Phenylring ein bis drei Substituenten, ausgewählt aus $C_1$-$C_4$-Alkoxy, Halogen, $C_1$-$C_4$-Alkyl oder Nitro tragen können;

Phenyl ggf. substituiert mit bis zu drei Substituenten, ausgewählt aus $C_1$-$C_4$-Alkoxy, Halogen, $C_1$-$C_4$-Alkyl oder Nitro; $C_1$-$C_5$-Alkoxy, ggf. substituiert mit Halogen, $C_1$-$C_4$-Alkoxy, Cyano, $C_1$-$C_4$-Dialkylamino, $C_1$-$C_4$-Thioalkyl, Phenyl, Phenoxy, Thiophenyl, Aminophenyl, $C_1$-$C_4$-Alkylaminophenyl, wobei die fünf letztgenannten Substituenten im Phenylring ein bis drei Substituenten, ausgewählt aus $C_1$-$C_4$-Alkoxy, Halogen, $C_1$-$C_4$-Alkyl oder Nitro tragen können;

$C_1$-$C_5$-Thioalkyl, ggf. halogensubstituiert;

ein fünf -oder sechsgliedriger gesättigter Heterocyclus mit ein bis zwei Heteroatomen aus der Gruppe Stickstoff, Sauerstoff oder Schwefel oder ein fünf- oder sechsgliedriger aromatischer Heterocyclus, mit ein bis drei Heteroatomen aus der Gruppe Stickstoff, Sauerstoff oder Schwefel, jeweils ggf. substituiert mit ein bis drei Substituenten ausgewählt aus: $C_1$-$C_4$-Alkoxy, Halogen, $C_1$-$C_4$-Alkyl, Cyano, Hydroxy, Amino, $C_1$-$C_4$-Alkylamino, $C_1$-$C_4$-Dialkylamino, $C_1$-$C_4$-Alkoxycarbonyl oder Nitro;

Hydroxy;

eine Gruppe $OC(O)R^{14}$;

Amino;

eine Gruppe $NR^{15}R^{16}$;

Halogen;

Cyano;
Nitro;
Carboxyl;
eine Gruppe $R^7SO_2$-;
eine Gruppe -C(O)$R^8$;
eine Gruppe -C(Y)$R^9$, wobei Y für Sauerstoff oder eine Gruppe $NR^{13}$ steht;
eine Gruppe -C($VR^{10}$ $WR^{11}$)$R^{12}$, wobei V und W unabhängig voneinander O oder S bedeuten;

$R^7$ $C_1$-$C_8$-Alkyl, $C_1$-$C_8$-Halogenalkyl, Phenyl, $C_1$-$C_8$-Monoalkyl- oder $C_1$-$C_8$-Dialkylamino, $C_5$-$C_8$-Cycloalkylamino;

$R^8$ Hydroxy, Amino, $C_1$-$C_8$-Monoalkyl- oder $C_1$-$C_8$-Dialkylamino, $C_5$-$C_8$-Cycloalkylamino, $C_1$-$C_8$-Alkoxy;

$R^9$ Wasserstoff, $C_1$-$C_8$-Alkyl, $C_3$-$C_8$-Cycloalkyl, $C_1$-$C_8$-Halogenalkyl, Phenyl, Heteroaryl;

$R^{10}$, $R^{11}$ $C_1$-$C_8$-Alkyl, $C_1$-$C_6$-Halogenalkyl, $C_1$-$C_4$-Alkoxy($C_1$-$C_6$-)alkyl oder $R^{10}$ und $R^{11}$ bilden zusammen eine Di-, Tri-, oder Tetramethylenkette, die ggf. durch ein bis zwei $C_1$-$C_8$-Alkyl-, $C_1$-$C_6$-Halogenalkyl-, $C_1$-$C_4$-Alkoxy($C_1$-$C_6$-)alkylgruppen oder eine Oxogruppe substituiert ist;

$R^{12}$ Wasserstoff, $C_1$-$C_8$-Alkyl, $C_3$-$C_8$-Cycloalkyl, $C_1$-$C_8$-Halogenalkyl, Phenyl, Heteroaryl;

$R^{13}$ $C_1$-$C_8$-Alkyl, $C_3$-$C_8$-Cycloalkyl, $C_1$-$C_4$-Alkoxy($C_1$-$C_6$-)alkyl, $C_1$-$C_8$-Aminoalkyl, Phenyl, Aryl($C_1$-$C_6$-)alkyl, Heteroaryl, Hydroxy, ggf. substituiertes $C_6$-$C_{10}$-Aryloxy, ggf. substituiertes $C_1$-$C_8$-Alkoxy, Amino, $C_1$-$C_8$-Monoalkyl- oder $C_1$-$C_8$-Dialkylamino, $C_5$-$C_8$-Cycloalkylamino, ggf substituiertes $C_6$-$C_{10}$-Arylamino;

$R^{14}$ $C_1$-$C_5$-Alkyl, ggf. subst. Phenyl, $C_1$-$C_5$-Alkoxy, $C_1$-$C_5$-Halogenalkoxy, Benzyloxy, Amino, Alkylamino, Dialkylamino, ein gesättigter über N gebundener fünf- oder sechsgliedriger Heterocyclus;

$R^{15}$ Wasserstoff, $C_1$-$C_5$-Alkyl, ggf. substituiertes Phenyl;

$R^{16}$ Wasserstoff, $C_1$-$C_5$-Alkyl, ggf. substituiertes Phenyl, eine Gruppe -C(O)$R^{14}$ oder $R^{16}$ bildet gemeinsam mit $R^{15}$ eine vier- oder fünfgliedrige Methylenkette, die ihrerseits mit ein bis zwei Methylgruppen substituiert sein kann und in der eine Methylengruppe durch Sauerstoff ersetzt sein kann;

$R^1$ ferner:
Phenyl, in dem zwei direkt benachbarte Positionen über eine Tri- oder Tetramethylengruppe, welche ihrerseits ein bis drei $C_1$-$C_3$-Alkyl-, $C_1$-$C_2$-Alkoxy- und/oder Halogensubstituenten tragen kann, miteinander verbunden sind und die übrigen Positionen unsubstituiert sind; oder
Phenyl, das über die ortho-Position mit $R^2$ verbunden ist zu einer Kette aus ein bis vier Methylengruppen oder ein bis drei Methylengruppen und einem Sauerstoffatom, wobei jede Methylengruppen ein oder zwei $C_1$-$C_3$-Alkylreste tragen kann.

**3.** Naphthyridine gemäß Anspruch 2, wobei $R^1$ einen Phenylrest bedeutet, der in ortho-Position substituiert ist.

**4.** Naphthyridine gemäß den Ansprüchen 2 und 3, wobei der ortho-Substituent am Phenylring die folgende Bedeutung hat: $C_1$-$C_5$-Alkyl, $C_3$-$C_6$-Cycloalkyl, $C_1$-$C_4$-Alkoxy, Chlor oder Fluor.

**5.** Naphthyridine gemäß Anspruch 1, in der $R^1$ unsubstituiertes Phenyl und $R^2$ einen der in Anspruch 1 genannten organischen Rest bedeuten.

**6.** Naphthyridine der Formel I gemäß Anspruch 1, in der die Substituenten die folgende Bedeutung haben:
$R^1$
Phenyl, substituiert mit bis zu vier Substituenten ausgewählt aus:
$C_1$-$C_8$-Alkyl, ggf. substituiert mit Halogen, $C_1$-$C_4$-Alkoxy, $C_1$-$C_4$-Thioalkyl, Phenyl, Phenoxy, Thiophenyl, wobei die drei letztgenannten Substituenten im Phenylring ein bis drei Substituenten, ausgewählt aus $C_1$-$C_4$-Alkoxy, Halogen, $C_1$-$C_4$-Alkyl oder Nitro tragen können;
$C_3$-$C_8$-Cycloalkyl, ggf. substituiert mit Halogen, $C_1$-$C_4$-Alkoxy, $C_1$-$C_4$-Thioalkyl, Phenyl, Phenoxy, Thiophenyl, wobei die drei letztgenannten Substituenten im Phenylring ein bis drei Substituenten, ausgewählt aus $C_1$-$C_4$-Alkoxy, Halogen, $C_1$-$C_4$-Alkyl oder Nitro tragen können;
Phenyl, ggf. substituiert mit bis zu drei Substituenten, ausgewählt aus $C_1$-$C_4$-Alkoxy, Halogen, $C_1$-$C_4$-Alkyl oder Nitro;
$C_1$-$C_5$-Alkoxy, ggf. substituiert mit Halogen, $C_1$-$C_4$-Alkoxy, Cyano, $C_1$-$C_4$-Dialkylamino, $C_1$-$C_4$-Thioalkyl,

Phenyl, Phenoxy, Thiophenyl, wobei die 3 letztgenannten Substituenten im Phenylring ein bis drei Substituenten, ausgewählt aus $C_1$-$C_4$-Alkoxy, Halogen, $C_1$-$C_4$-Alkyl oder Nitro tragen können;
$C_1$-$C_5$-Thioalkyl, $C_1$-$C_4$-Halogenthioalkyl;
eine Gruppe $OC(O)R^{14}$;
eine Gruppe $NR^{15}R^{16}$;
Halogen;
Cyano;
Nitro;
Carboxyl;
eine Gruppe $R^7SO_2$-;
eine Gruppe -$C(O)R^8$;
eine Gruppe -$C(Y)R^9$, wobei Y für Sauerstoff oder eine Gruppe $NR^{13}$ steht; oder
eine Gruppe -C ($VR^{10}$ $WR^{11}$)$R^{12}$, wobei V und W unabhängig voneinander O oder S bedeuten;

$R^7$ $C_1$-$C_8$-Alkyl, $C_1$-$C_8$-Halogenalkyl, Phenyl;
$R^8$ $C_1$-$C_8$-Monoalkyl- oder $C_1$-$C_8$-Dialkylamino, $C_5$-$C_8$-Cycloalkylamino, $C_1$-$C_8$-Alkoxy;
$R^9$ Wasserstoff, $C_1$-$C_8$-Alkyl, $C_3$-$C_8$-Cycloalkyl, $C_1$-$C_8$-Halogenalkyl, Phenyl, Heteroaryl;
$R^{10}$, $R^{11}$ $C_1$-$C_8$-Alkyl oder $R^{10}$ und $R^{11}$ bilden gemeinsam eine Di-, Tri-, oder Tetramethylenkette, die ggf. durch ein bis zwei $C_1$-$C_8$-Alkyl-, $C_1$-$C_6$-Halogenalkyl-, $C_1$-$C_4$-Alkoxy($C_1$-$C_6$)-alkylgruppen oder eine Oxogruppe substituiert ist;
$R^{12}$ Wasserstoff, $C_1$-$C_8$-Alkyl, $C_3$-$C_8$-Cycloalkyl, $C_1$-$C_8$-Halogenalkyl, Phenyl, Heteroaryl;
$R^{13}$ $C_1$-$C_8$-Alkyl, Phenyl, Aryl($C_1$-$C_6$-)alkyl, ggf. substituiertes $C_6$-$C_{10}$-Aryloxy, ggf. substituiertes $C_1$-$C_8$-Alkoxy;
$R^{14}$ $C_1$-$C_5$-Alkyl, ggf. substituiertes Phenyl, $C_1$-$C_5$-Alkoxy, $C_1$-$C_5$-Halogenalkoxy, Benzyloxy, Amino, Alkylamino, Dialkylamino, ein gesättigter über N gebundener fünf- oder sechsgliedriger Heterocyclus;
$R^{15}$ Wasserstoff, $C_1$-$C_5$-Alkyl, ggf. substituiertes Phenyl;
$R^{16}$ Wasserstoff, $C_1$-$C_5$-Alkyl, ggf. substituiertes Phenyl, eine Gruppe -$C(O)R^{14}$ oder $R^{16}$ bildet gemeinsam mit $R^{15}$ eine vier- oder fünfgliedrige Methylenkette, die ihrerseits mit ein bis zwei Methylgruppen substituiert sein kann und in der eine Methylengruppe durch Sauerstoff ersetzt sein kann;

$R^1$ ferner:
Phenyl, in dem zwei direkt benachbarte Positionen über eine Tri- oder Tetramethylengruppe, welche ihrerseits ein bis drei $C_1$-$C_3$-Alkyl-, $C_1$-$C_2$-Alkoxy- und/oder Halogensubstituenten tragen kann, miteinander verbunden sind und die übrigen Positionen unsubstituiert oder durch einen der unter (c) aufgeführten Phenylsubstituenten substituiert sind;
$R^2$
Wasserstoff, $C_1$-$C_8$-Alkyl, $C_1$-$C_8$-Halogenalkyl, $C_3$-$C_8$-Cycloalkyl, ggf substituiertes Phenyl, Benzyl;
$R^3$-$R^6$
Wasserstoff, $C_1$-$C_8$-Alkyl, $C_1$-$C_8$-Halogenalkyl, $C_3$-$C_8$-Cycloalkyl, ggf substituiertes Phenyl, Benzyl, ggf. substituiertes über einen Kohlenstoff verknüpftes fünf- oder sechsgliedriges Heteroaryl, Cyano, Nitro, Carboxyl, Sulfonylmethyl, $C_1$-$C_4$-Alkoxycarbonyl, Carbamoyl, Alkylcarbamoyl, Dialkylcarbamoyl, $C_1$-$C_4$-Alkanoyl oder Benzoyl.

**7.** Naphthyridine der Formel I gemäß Anspruch 1, in der die Sübstituenten $R^2$-$R^6$ die in Anspruch 1 genannte Bedeutung haben und $R^1$ wie folgt definiert ist:
ein Heteroaromat, ausgewählt aus der Gruppe aus 2-Thienyl, 2-Benzothienyl, 3-Thienyl, 2-Furanyl, 3-Furanyl, 2-Benzofuranyl, 2-Pyrrolyl, 3-Pyrrol, 2-Thiazolyl, 4-Thiazolyl, 5-Thiazolyl, 2-Oxazolyl, 4-Oxazolyl, 5-Oxazolyl, 2-Pyridyl, 3-Pyridyl, 4-Pyridyl, 2-Pyrimidinyl, 4-Pyrimidinyl, 5-Pyrimidinyl, 2-Benzothiazolyl, 2-Benzooxazolyl, wobei die Heteroaromaten ein bis zwei der folgenden Substituenten tragen können:
$C_1$-$C_8$-Alkyl, $C_3$-$C_8$-Cycloalkyl, $C_1$-$C_8$-Halogenalkyl, Phenyl, $C_1$-$C_5$-Alkoxy, $C_1$-$C_3$-Halogenalkoxy, Halogen, Cyano, Nitro, Carboxyl, Sulfonylmethyl, $C_1$-$C_4$-Alkoxycarbonyl, Carbamoyl, $C_1$-$C_4$-Alkylcarbamoyl, Di-($C_1$-$C_4$)-alkylcarbamoyl.

**8.** Herbizides Mittel, enthaltend mindestens ein substituiertes Naphthyridin der Formel I gemäß Anspruch 1 und übliche inerte Trägerstoffe.

9. Herbizides Mittel, enthaltend mindestens ein substituiertes Naphthyridin der Formel I gemäß Anspruch 6 und übliche inerte Trägerstoffe.

10. Verfahren zur Bekämpfung unerwünschten Pflanzenwuchses, dadurch gekennzeichnet, daß man eine herbizid wirksame Menge eines substituierten Naphthyridins der Formel I

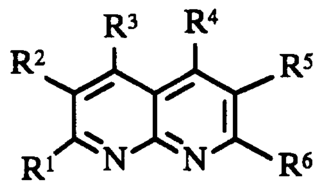

I

in der die Substituenten folgende Bedeutung haben:

$R^1$

(a) ein ggf. substituierter fünfgliedriger Heteroaromat mit ein bis zwei Heteroatomen, ausgewählt aus Stickstoff, Sauerstoff oder Schwefel;

(b) ein ggf. substituierter sechsgliedriger Heteroaromat enthaltend ein bis zwei Stickstoffatome;

(c) Phenyl, substituiert mit ein bis vier Substituenten ausgewählt aus der Gruppe $C_1$-$C_8$-Alkyl, $C_2$-$C_8$-Alkenyl, $C_2$-$C_8$-Alkinyl,$C_3$-$C_8$-Cycloalkyl, Phenyl, $C_1$-$C_5$-Alkoxy, $C_1$-$C_5$-Thioalkyl, ein fünf -oder sechsgliedriger gesättigter Heterocyclus, ein fünf -oder sechsgliedriger aromatischer Heterocyclus, wobei alle voranstehend genannten Substituenten jeweils noch selber ein- bis dreimal substituiert sein können, Hydroxy, eine Gruppe $OC(O)R^{14}$, Amino, eine Gruppe $NR^{15}R^{16}$, Halogen, Cyano, Nitro, Carboxyl, eine Gruppe $R^7SO_2$-, -$C(O)R^8$, -$C(Y)R^9$, wobei Y für Sauerstoff oder eine Gruppe $NR^{13}$ steht, -$C(VR^{10}WR^{11})R^{12}$, wobei V und W unabhängig voneinander für O oder S stehen;

| | |
|---|---|
| $R^7$ | $C_1$-$C_8$-Alkyl, $C_3$-$C_8$-Cycloalkyl, $C_1$-$C_8$-Halogenalkyl, $C_1$-$C_5$-Alkoxy, Phenyl, Naphthyl, Aryl($C_1$-$C_6$-)alkyl, Heteroaryl, Hydroxy, Amino, $C_1$-$C_8$-Monoalkyl- oder $C_1$-$C_8$-Dialkylamino, $C_5$-$C_8$-Cycloalkylamino; |
| $R^8$ | Hydroxy, Amino, $C_1$-$C_8$-Monoalkyl- oder $C_1$-$C_8$-Dialkylamino, $C_5$-$C_8$-Cycloalkylamino, $C_1$-$C_6$-Alkylthio, $C_1$-$C_8$-Alkoxy; |
| $R^9$ | Wasserstoff, $C_1$-$C_8$-Alkyl, $C_3$-$C_8$-Cycloalkyl, $C_1$-$C_8$-Halogenalkyl, $C_1$-$C_4$-Alkoxy($C_1$-$C_6$-)alkyl, $C_1$-$C_8$-Aminoalkyl, Phenyl, Aryl($C_1$-$C_6$-)alkyl, Heteroaryl; |
| $R^{10}$, $R^{11}$ | $C_1$-$C_8$-Alkyl, $C_1$-$C_6$-Halogenalkyl, $C_1$-$C_4$-Alkoxy-($C_1$-$C_6$-)alkyl oder $R^{10}$ und $R^{11}$ bilden zusammen eine Di-, Tri-, oder Tetramethylenkette die ggf. durch ein bis zwei $C_1$-$C_8$-Alkyl-, $C_1$-$C_6$-Halogenalkyl-, $C_1$-$C_4$-Alkoxy($C_1$-$C_6$-)alkylgruppen oder durch eine Oxogruppe substituiert ist; |
| $R^{12}$ | Wasserstoff, $C_1$-$C_8$-Alkyl, $C_3$-$C_8$-Cycloalkyl, $C_1$-$C_8$-Halogenalkyl, $C_1$-$C_4$-Alkoxy($C_1$-$C_6$-)alkyl, $C_1$-$C_8$-Aminoalkyl, Phenyl, Aryl($C_1$-$C_6$-)alkyl, Heteroaryl; |
| $R^{13}$ | $C_1$-$C_8$-Alkyl, $C_2$-$C_8$-Alkenyl, $C_3$-$C_8$-Cycloalkyl, $C_1$-$C_8$-Halogenalkyl, $C_1$-$C_4$-Alkoxy($C_1$-$C_6$-)alkyl, $C_1$-$C_8$-Aminoalkyl, Phenyl, Aryl($C_1$-$C_6$-)alkyl, Heteroaryl, Hydroxy, ggf. substituiertes $C_6$-$C_{10}$-Aryloxy, ggf. substituiertes $C_1$-$C_8$-Alkoxy, Amino, $C_1$-$C_8$-Monoalkyl- oder $C_1$-$C_8$-Dialkylamino, $C_5$-$C_8$-Cycloalkylamino, ggf. substituiertes $C_6$-$C_{10}$-Arylamino; |
| $R^{14}$ | $C_1$-$C_5$-Alkyl, ggf. substituiertes Phenyl, $C_1$-$C_5$-Alkoxy, $C_1$-$C_5$-Halogenalkoxy, Benzyloxy, Amino, Alkylamino, Dialkylamino, ein gesättigter über N gebundener fünf- oder sechsgliedriger Heterocyclus, der noch ein weiteres Heteroatom N oder O enthalten kann; |
| $R^{15}$ | Wasserstoff, $C_1$-$C_5$-Alkyl, ggf. substituiertes Phenyl; |
| $R^{16}$ | Wasserstoff, $C_1$-$C_5$-Alkyl, ggf. substituiertes Phenyl, eine Gruppe -$C(O)R^{14}$ oder $R^{16}$ bildet gemeinsam mit $R^{15}$ eine vier- oder fünfgliedrige Methylenkette, die ihrerseits mit ein oder zwei Methylgruppen substituiert sein kann und in der eine Methylengruppe durch Sauerstoff ersetzt sein kann; |

(d) Phenyl, in dem zwei direkt benachbarte Positionen über eine Tri- oder Tetramethylengruppe, welche ihrerseits ein bis drei $C_1$-$C_3$-Alkyl-, $C_1$-$C_2$-Alkoxy- und/oder Halogensubstituenten tragen kann, miteinander verbunden sind und die übrigen Positionen unsubstituiert oder durch einen der unter (c) aufgeführten Phenylsubstituenten substituiert sind;

(e) Phenyl, das über die ortho-Position mit $R^2$ verbunden ist zu einer Kette aus ein bis vier Methylengruppen oder ein bis drei Methylengruppen und einem Sauerstoffatom, wobei jede Methylengruppen ein oder zwei $C_1$-$C_3$-Alkylreste tragen kann;

$R^2$-$R^6$

Wasserstoff, $C_1$-$C_8$-Alkyl, $C_2$-$C_8$-Alkenyl, $C_3$-$C_8$-Cycloalkyl, $C_1$-$C_8$-Halogenalkyl, $C_1$-$C_4$-Alkoxy($C_1$-$C_6$)-alkyl, $C_1$-$C_8$-Aminoalkyl, ggf. substituiertes Phenyl, Benzyl, ggf substituiertes über einen Kohlenstoff verknüpftes fünf- oder sechsgliedriges Heteroaryl, Cyano, Nitro, Carboxyl, Sulfonylmethyl, $C_1$-$C_4$-Alkoxycarbonyl, Carbamoyl, $C_1$-$C_8$-Alkylcarbamoyl, Dialkylcarbamoyl, $C_1$-$C_4$-Alkanoyl, Benzoyl oder $R^4$ und $R^5$ oder $R^5$ und $R^6$ bilden gemeinsam eine Kette $CH_2$-$(CH_2)_n$-$CH_2$ mit n = 1 bis 3 oder einen unkondensierten aromatischen Ring;

auf die Pflanzen und/oder deren Lebensraum einwirken läßt.

**11.** Mittel gegen Schadpilze, enthaltend übliche inerte Zusatzstoffe und mindestens ein substituiertes Naphthyridin der Formel I gemäß Anspruch 1.

**12.** Verfahren zur Bekämpfung von tierischen Schädlingen oder Schadpilzen, dadurch gekennzeichnet, daß man die Schädlinge oder Schadpilze, deren Lebensraum oder die von ihnen freizuhaltenden Pflanzen, Flächen, Materialien oder Räume mit einer wirksamen Menge einer Verbindung der Formel I gemäß Anspruch 1 behandelt.